# EUROPEAN PATENT SPECIFICATION

(11) **EP 1 294 915 B1**
(45) Date of publication and mention of the grant of the patent: **07.09.2005**
(21) Application number: 01949376.6
(22) Date of filing: 31.05.2001
(51) Int. Cl.: C12N 15/82, C07K 14/415, C12N 15/29, C12N 5/14, A01H 5/10

(54) **HETEROLOGOUS GENE EXPRESSION IN PLANTS**
HETEROLOGE GENEXPRESSION IN PFLANZEN
EXPRESSION D'UN GENE HETEROLOGUE DANS DES VEGETAUX

(30) Priority: 29.06.2000 EP 00202278
(43) Date of publication of application: 26.03.2003
(73) Proprietor: Vlaams Interuniversitair Instituut voor Biotechnologie vzw., 9052 Zwijnaarde (BE)
(72) Inventor: ANGENON, Geert, B-9000 Gent (BE); DE JAEGER, Geert, B-9940 Evergem (BE); GOOSSENS, Alain, B-9160 Lokeren (BE); DEPICKER, Anna, B-9820 Merelbeke (BE)
(74) Representative: Brants, Johan P.E.
(86) International application number: PCT/EP2001/006298
(87) International publication number: WO 2002/000899

(56) References cited:
- EP-A- 0 723 019
- GOOSSENS ALAIN ET AL: "The arcelin-5 gene of Phaseolus vulgaris directs high seed-specific expression in transgenic Phaseolus acutifolius and Arabidopsis plants." PLANT PHYSIOLOGY (ROCKVILLE), vol. 120, no. 4, August 1999 (1999-08), pages 1095-1104, XP002155235 ISSN: 0032-0889 cited in the application
- FIEDLER U ET AL: "HIGH-LEVEL PRODUCTION AND LONG-TERM STORAGE OF ENGINEERED ANTIBODIES IN TRANSGENIC TOBACCO SEEDS" BIO/TECHNOLOGY,US,NATURE PUBLISHING CO. NEW YORK, vol. 13, no. 10, 1 October 1995 (1995-10-01), pages 1090-1093, XP002033957 ISSN: 0733-222X
- KREBBERS E ET AL: "DETERMINATION OF THE PROCESSING SITES OF AN ARABIDOPSIS 2S ALBUMIN AND CHARACTERIZATION OF THE COMPLETE GENE FAMILY" PLANT PHYSIOLOGY (BETHESDA), vol. 87, no. 4, 1988, pages 859-866, XP000974809 ISSN: 0032-0889 cited in the application
- GOOSSENS ALAIN ET AL: "Isolation and characterisation of arcelin-5 proteins and cDNAs." EUROPEAN JOURNAL OF BIOCHEMISTRY, vol. 225, no. 3, 1994, pages 787-795, XP000974389 ISSN: 0014-2956 cited in the application

## Description

The present invention relates to heterologous gene expression in plants. More specifically, the invention relates to high expression of heterologous proteins in seeds, by incorporating the gene between seed specific sequences. Preferably, said heterologous protein is a single-chain antibody variable fragment (scFv).

The ability to clone and produce a wide range of proteins from diverse sources became feasible with the advent of recombinant technology. The selection of expression hosts for commercial production of heterologous proteins is based on the economics of the production technique, such as the fermentation cost, on the cost of the purification and on the ability of the host to accomplish the post-translational modifications needed for full biological activity of the recombinant protein.

Although in many cases, prokaryotic cells such as *Escherichia coli* or simple eukaryotic cells such as the yeast *Saccharomyces cerevisiae* are the host cells of choice, these systems are not sufficient in all cases and problems can be encountered both in yield and activity of the protein produced. Alternative systems such as plant cells, mammalian cells and insect cells may solve the problem of biological activity, but suffer from a high fermentation cost and a low yield.

Transgenic plants can produce several types of heterologous polypeptides, comprising antibodies (ab's) and antibody fragments (Whitelam *et al*., 1993; Goddijn and Pen, 1995; Hemming, 1995). Antibodies and antibody fragments are interesting from an industrial point of view: they can be produced against nearly every type of organic molecule and are binding this antigen in a very specific way. However, the major drawback is the production cost. Plants and plant cells are an interesting alternative for the production of these molecules, and other polypeptides that are difficult to produce in other prokaryotic or eukaryotic cells.

US5804694 describes the commercial production of β-glucuronidase in plants, by placing the β-glucuronidase gene after an ubiquitin promoter. With this construction, 0.1% of the total extracted protein is β-glucuronidase. By targeting the heterologous protein to the endoplasmic reticulum, the accumulation can be improved, especially for ab's and ab fragments. Using the cauliflower mosaic virus 35S promoter resulted in expression level of single chain Fv (scFv) antibodies up to 4 - 6.8% of total soluble protein in leaves (Fiedler *et al.,* 1997).

A lot of the efforts for production of proteins in plants have been focused on seeds. Indeed, seeds, especially those of legumes and cereals, contain large quantities of protein; these are mainly storage proteins, which can form up to 7-15% of the dry weight for cereals, and up to 20-40% for legumes. Moreover, those storage proteins are limited in number, and some of them can be responsible for up to 20% of the total protein content in seed (Vitale & Bollini, 1995), which may have important advantages for the purification process. In that respect, the promoters coding for the storage proteins have been considered as ideal tools to obtain high expression levels of proteins in seed (Fiedler *et al*., 1997)

US5504200 discloses the use of the phaseolin promoter for the expression of heterologous genes in plants and plant cells. WO9113993 describes the expression of animal genes, or the gene from brazil nut 2S storage protein, using a promoter selected from the group consisting of the phaseolin promoter, the α'-subunit of β-conglycinin promoter and the β-zein promoter. The gene is linked to a poly-A signal selected from the group consisting of phaseolin poly-A signal and animal poly-A signal.

However, none of these systems leads to high heterologous protein expression. WO9729200 describes a seed specific expression level of 1.9% of heterologous protein on total soluble protein, using the specific legumin B4 promoter. Further improvement of the expression cassettes lead to an expression of 3-4% of scFv antibodies in ripe tobacco seeds (Fiedler *et al*., 1997)

Recently, the arcelin 5I gene (*arc5I*) of *Phaseolus vulgaris* was isolated and cloned. This gene is responsible for the production of the seed storage protein Arcelin 5a (ARC5a) that accumulates in wild type plants up to 24-32% of the total protein content of the seed (Goossens *et al*., 1994; Goossens *et al*., 1995). Expression of the gene in *Arabidopsis thaliana* and *Phaseolus acutifolius* indicated that the seed storage protein ARC5a could be expressed up to 15%, respectively 25% of the total soluble protein (Goossens *et al*., 1999), when the own promoter and expression signals were used. However, no evidence was shown that the promoter could give efficient expression with other proteins. Surprisingly, we found that the use of the arcelin 5I promoter or the phaseolin promoter, in combination with the arcelin 5l leader sequence or the Tobacco Mosaic Virus (TMV) omega leader and the arcelin 5l 3' sequence could result in an expression level of heterologous protein as high as 12% of the total seed protein, which is far higher than known in the prior art.

It is a first aspect of the invention to provide a seed preferred expression cassette having gene regulatory elements comprising
- the arcelin promoter comprising the sequence shown in SEQ ID NO: 1
- the arcelin 5I leader shown in SEQ ID NO:2
- the 252 storage albumin signal peptide shown in SEQ ID NO:4
- the arcelin 5I 3' end comprising the sequence shown in SEQ ID NO:3, wherein
said heterologous gene of interest is placed between said leader sequence and said arcelin 5I 3' sequence.

Preferably, said gene of interest is fused to the sequence encoding the 2S2 storage albumin signal peptide. In one preferred embodiment, the gene of interest is a gene encoding a scFv antibody.

It is another aspect of the invention to provide a method to obtain seed preferred expression of a heterologous protein using the seed preferred expresion cassette of the invention at a level of at least 10%, preferably a level of at least 15%, 20%, 25%, 30%, 35% or 40 % of the total soluble seed protein, with the proviso that said heterologous protein is not an unmodified seed storage protein such as Arcelin 5a. Preferably, said heterologous protein is not an unmodified Arcelin, Phaseolin or Zein. Another preferred embodiment is a method according to the invention, whereby said heterologous protein is a scFv.

Still another aspect of the invention is a plant cell, transformed with an expression cassette according to the invention, or a transgenic plant comprising an expression cassette according to the invention. Indeed, the expression cassette can be incorporated and transformed to a plant cell or plant, using methods known to the person skilled in the art. Said methods include, but are not limited to *Agrobacterium* T-DNA mediated transformation, particle bombardment, electroporation and direct DNA uptake.

### Definitions

The following definitions are set forth to illustrate and define the meaning and scope of various terms used to describe the invention herein.

*Seed preferred expression* means that the expression preferably takes place in the seed, but does not exclude expression in other organs of the plant.

*Gene of interest* as used here means the coding sequence of a gene of which one wants to obtain seed preferred expression.

*Leader* as used here means the 5' end untranslated sequence.

*Signal peptide* indicates the initial function of the peptide in the 2S2 albumin storage protein but does not necessarily imply that the peptide has the same function and is processed in the same way when it is fused to the gene of interest.

*Heterologous protein* refers to any protein that can be expressed in seed but which is not an unmodified seed storage protein. In contrast, modified seed storage proteins (specific mutants, fusion proteins, improved seed storage proteins...) are part of the present invention and are thus included in said definition.

### Brief description of the figures

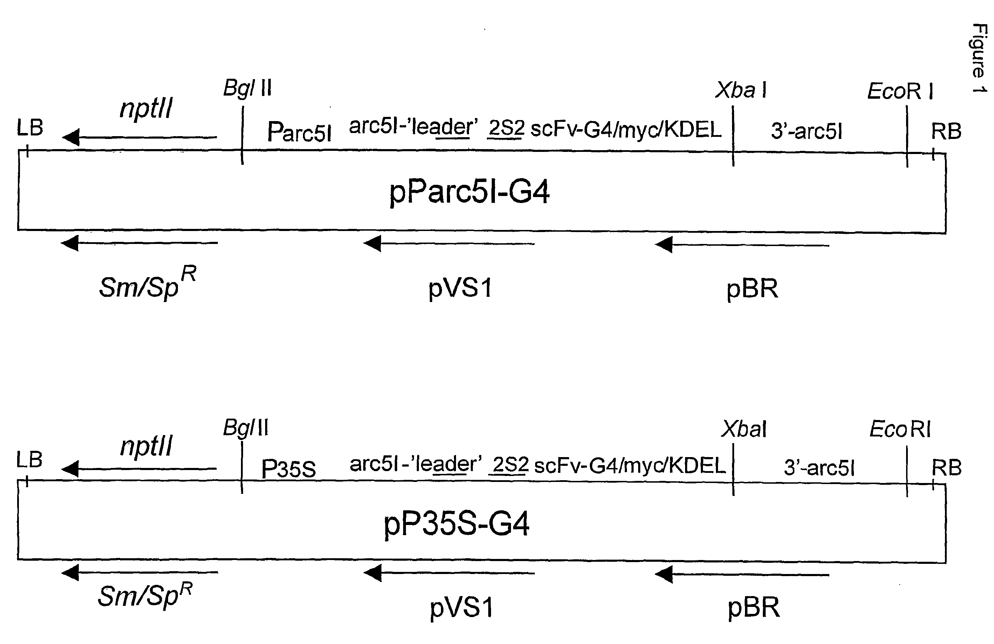

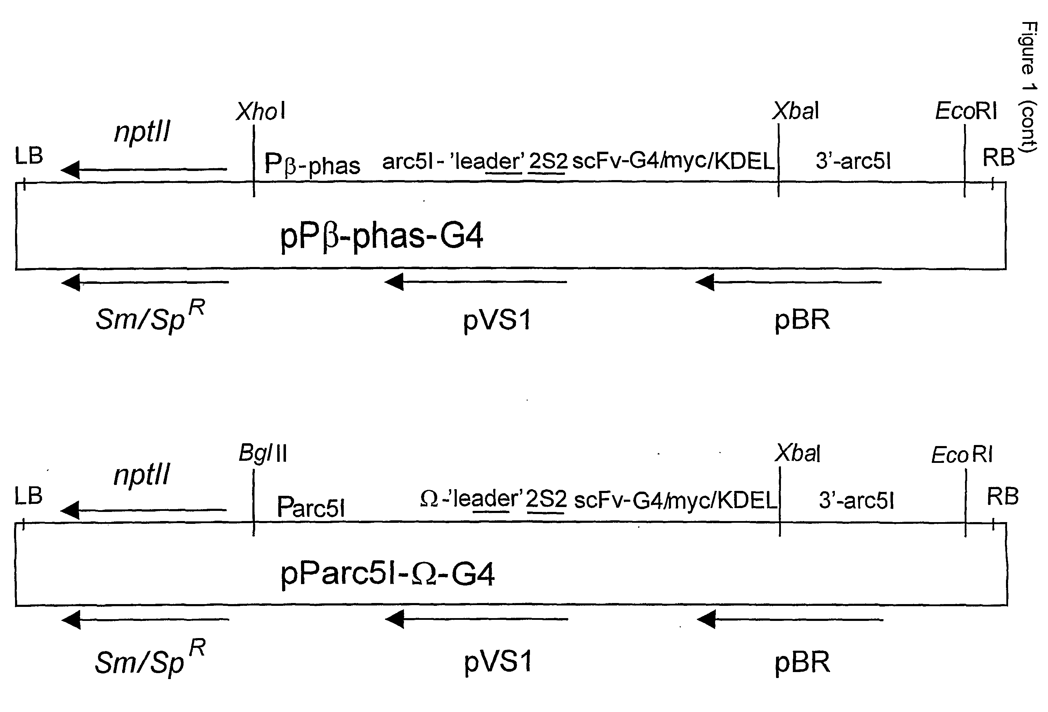

**Figure 1:** Overview of the T-DNA vectors for the evaluation of scFv production in seeds of *Arabidopsis thaliana*.
   LB and RB = left- en right border of the T-DNA
   pVS1 = plasmid insertion of *Pseudomonas aeruginosa* for vector stability and replication in *Agrobacterium tumefaciens.*
   pBR = ori of replication in *Escherichia coli*.
   *NptII* = selection marker *neomycine phosphotransferase II* under control of the *nos*-promoter and the *ocs* 3'-termination and poly-adenylation-signals.
   *Sm*/*SpR* = bacterial resistance gene for spectinomycin and streptomycin
**Figure 2:** Construction of pBluescript (2S2-G4)
**Figure 3:** Construction of patag5 (3'-arc5l)
**Figure 4:** Construction of pSP72 (Parc5l/ARC5a^{cs})
**Figure 5**: Amplification of DNA fragments for vector construction
**Figure 6:** Construction of pParc5l-G4. This vector has been, in accordance with the Budapest Treaty, deposited with the Belgian Coordinated Collections of Microorganisms-BCCM™ Laboratorium voor Moleculaire Biologie-Plasmidencollectie (LMBP), Universiteit Gent, K.L. Ledeganckstraat 35, B-9000 Gent, Belgium by Dr. Ann Depicker, Molenstraat 61, 9820 Merelbeke, Belgium (work: K.L. Ledeganckstraat 35, 9000 Gent, Belgium) and has accession number: LMBP 4128.
**Figure 7:** Construction of pParc5l-Ω-G4
**Figure 8:** Construction of pP35S-G4
**Figure 9:** Construction of pPβ-phas-G4
**Figure 10:** Results of scFv-G4 quantification in seed extracts by quantitative Western blot. 500 ng protein of A-, P-, and Ω-seed extracts and 2,5 µg protein of 35S- and Col O-extracts were loaded on a 10 % SDS-PAGE gel. G4 proteins were detected by monoclonal anti-*c*-*myc* antibody and anti-mouse antibody coupled to alkaline phosphatase. ColO = negative control.

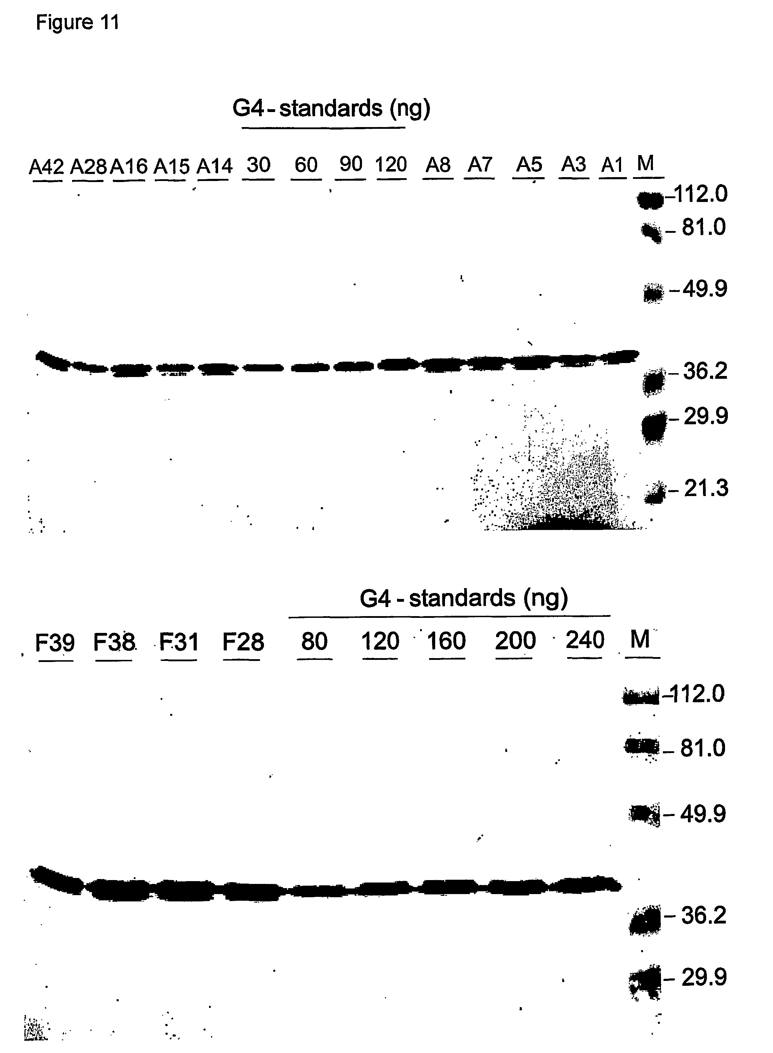

**Figure 11:** scFv-G4 quantification in seed extracts from floral dip transformants by quantitative Western blot. Results are shown from 10 segregating T2 seed stocks transformed with pParc5l-G4 (upper blot), and 4 segregating T2 seed stocks transformed with pPβ-phas-G4 (lower blot). We loaded 1 microgram seed protein for A1, A7, F28, F31, F38, and F39, 1.5 microgram for A3, A5, A8, A15, A22, and A42; and 2 microgram for A14 and A16 on a 10% SDS-PAGE gel. G4 proteins were detected by monoclonal anti-*c-myc* antibody and anti-mouse antibody coupled to alkaline phosphatase. M = molecular weight marker.

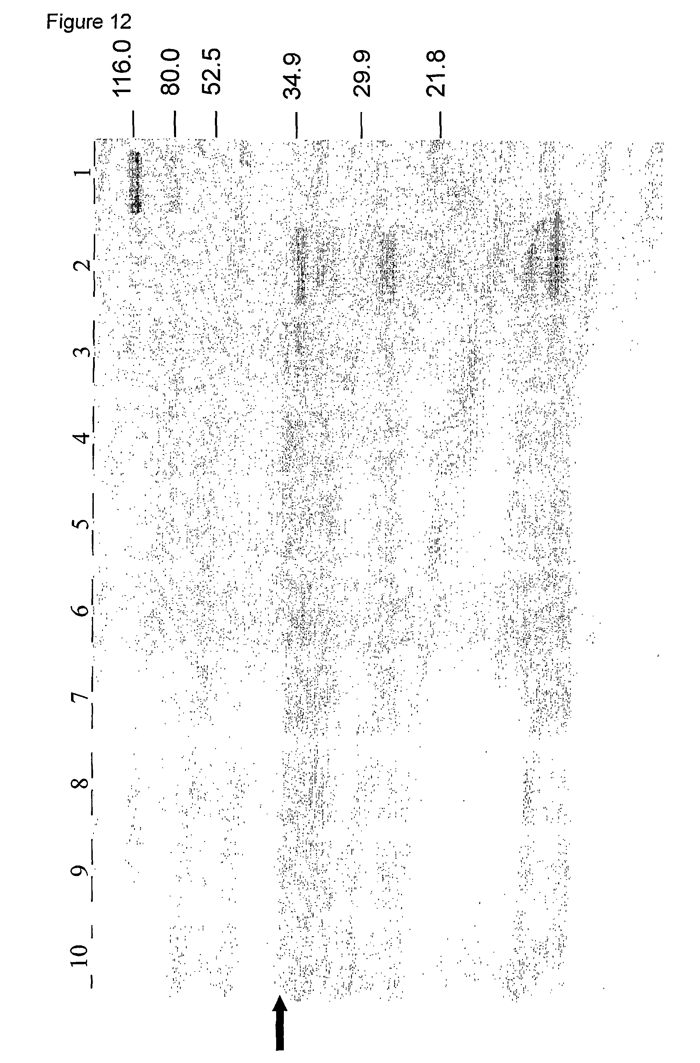

**Figure 12:** Coomassie blue stained SDS/page gel showing separated *Arabidopsis* seed proteins from transgenics F28 (lanes 3 and 7), F31 (lanes 4 and 8), F38 (lanes 5 and 9), F39 (lanes 6 and 10), transformed with pPβ-phas-G4 and from an untransformed control plant (lane 2). Lanes 3, 4, 5, and 6 contain 30 microgram protein and lanes 7, 8, 9, and 10 contain 20 microgram protein. The arrow indicates the recombinant scFv protein band. Lane 1 contains the molecular weight marker. On basis of the coomassie stained protein bands in separate lanes, Image master VDS software measured following G4 contents for each line: 9.9% for F28 (11.3% by Western blot), 15.4% for F31 (20.0% by Western blot), 16.0% for F38 (19.0% by Western blot), and 9.6% for F39 (12.0 % by Western blot).
**Figuur 13:** Schematic representation of the ELISA-test used to analyse the antigen binding activity of *ex planta*-extracted and *E. coli*-extracted scFv-proteins. (1) Coating of microtiter well with monoclonal antibody 9E10 binding to the *cmyc*-tag of the scFv; (2) co-incubation of several amounts of scFv from seed or *E. coli* with an excess of the antigen dihydroflavonole-4-reductase (DFR) from *Petunia hybrida*; detection of bound DFR with (3) polyclonal antiserum against DFR from rabbit and (4) polyclonal anti-rabbit serum coupled to alkaline phosphatase (AP).
**Figuur 14:** Results of the analysis of antigen-binding activity of seed- and *E.coli*-extracted scFv proteins by ELISA. The ELISA test (figure 12) was performed with different G4-concentrations (X-axis) in presence or absence (controls) of DFR-antigen. Y-axis represents the ELISA-signal (ΔFU/min).
**Figuur 15:** Construction of patag6 (3'-arc5l).

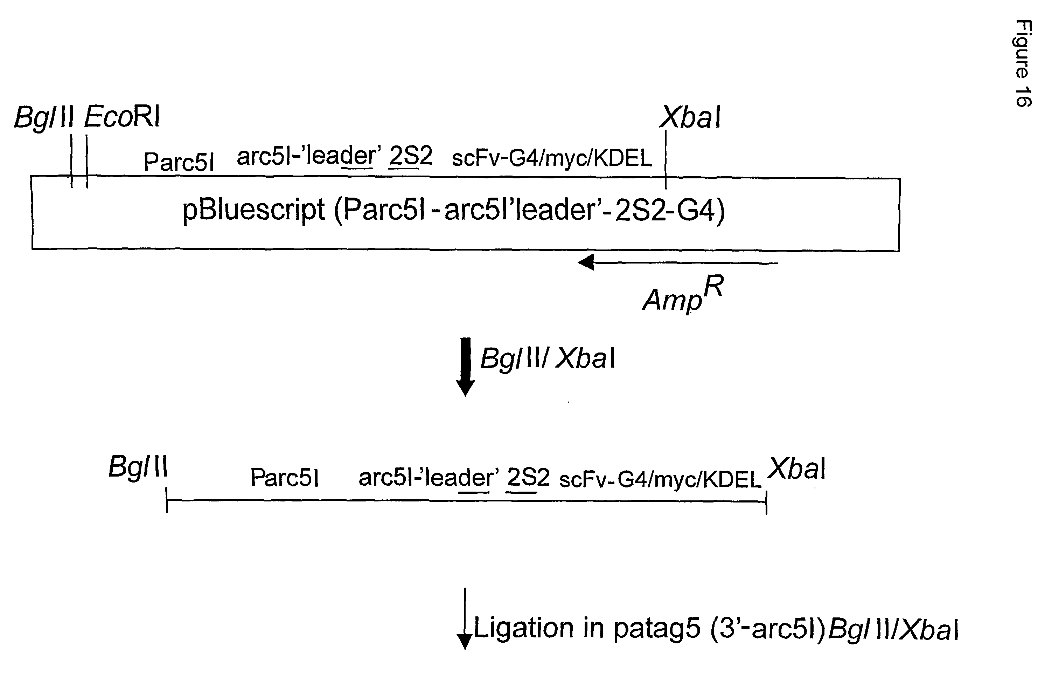

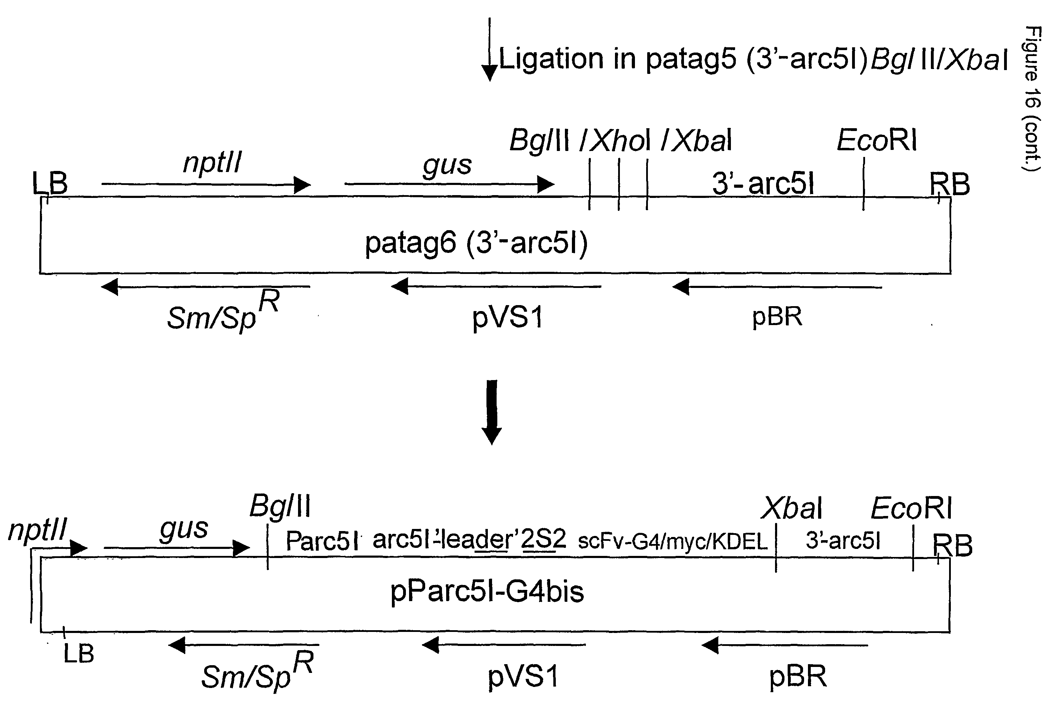

**Figure 16:** Construction of pParc5l-G4bis.

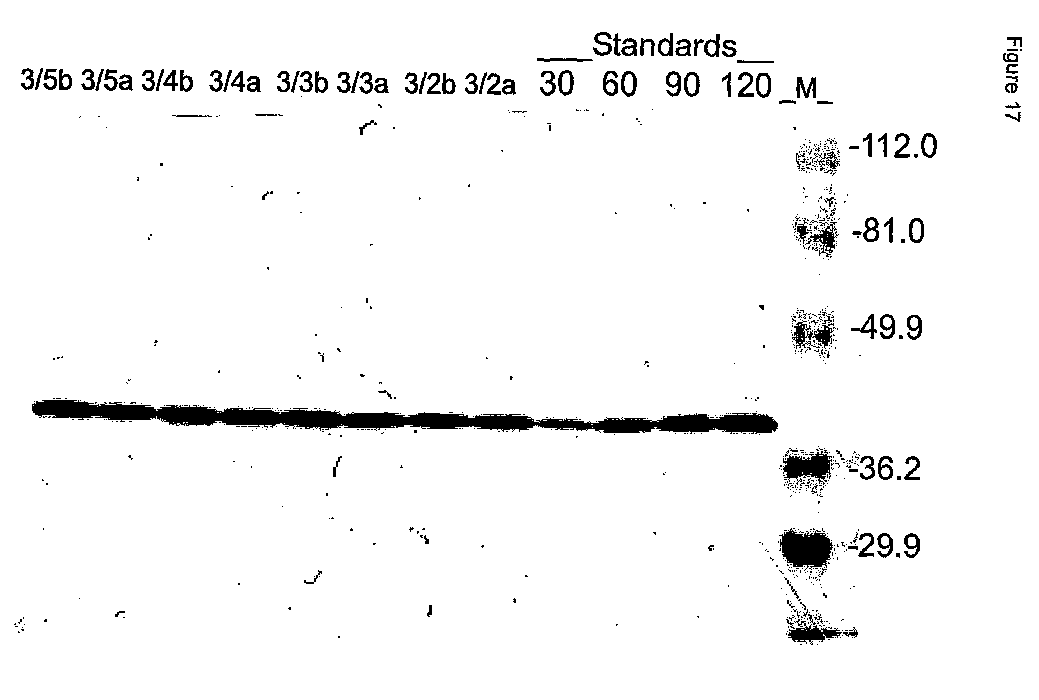

**Figure 17:** scFv-G4 quantification in different seeds (3/2, 3/3, 3/4, and 3/5) from a single transgenic Phaseolus acutifolius plant. From each seed extract, we loaded 3 (lanes a) or 4 (lanes b) microgram seed protein on a 10% SDS-PAGE gel. G4 proteins were detected by monoclonal anti-c-myc antibody and anti-mouse antibody coupled to alkaline phosphatase. M = molecular weight marker.

### Examples

### Example 1: Cloning of the T-DNA vectors for the evaluation of scFv production in Arabidopsis thaliana under control of the arc5l expression signals of Phaseolus vulgaris.

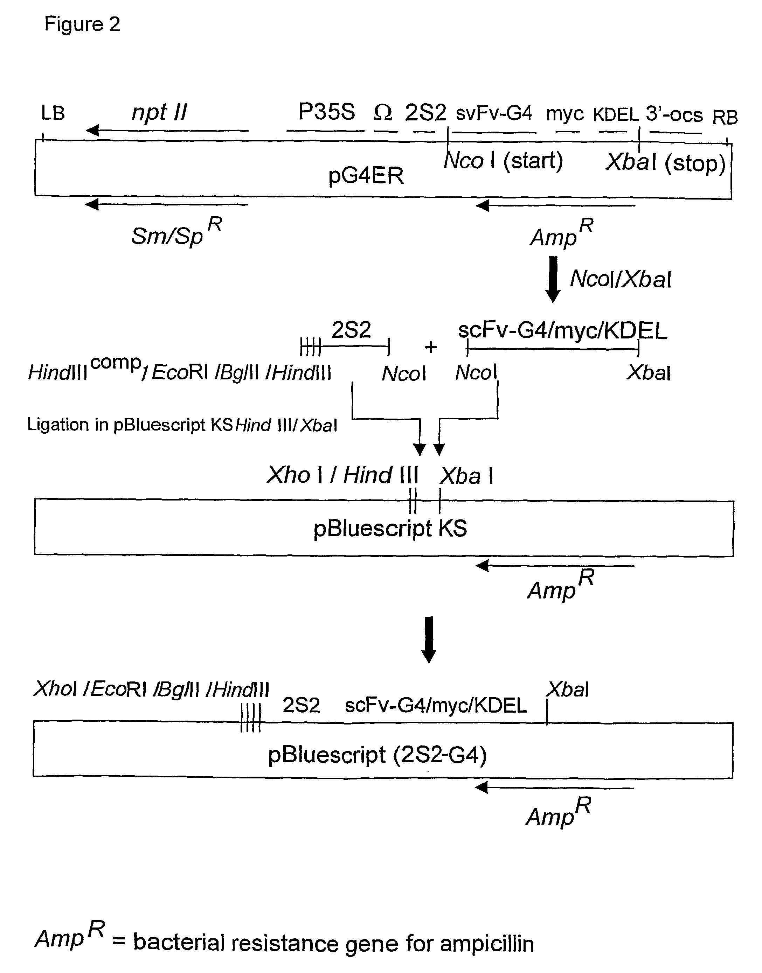

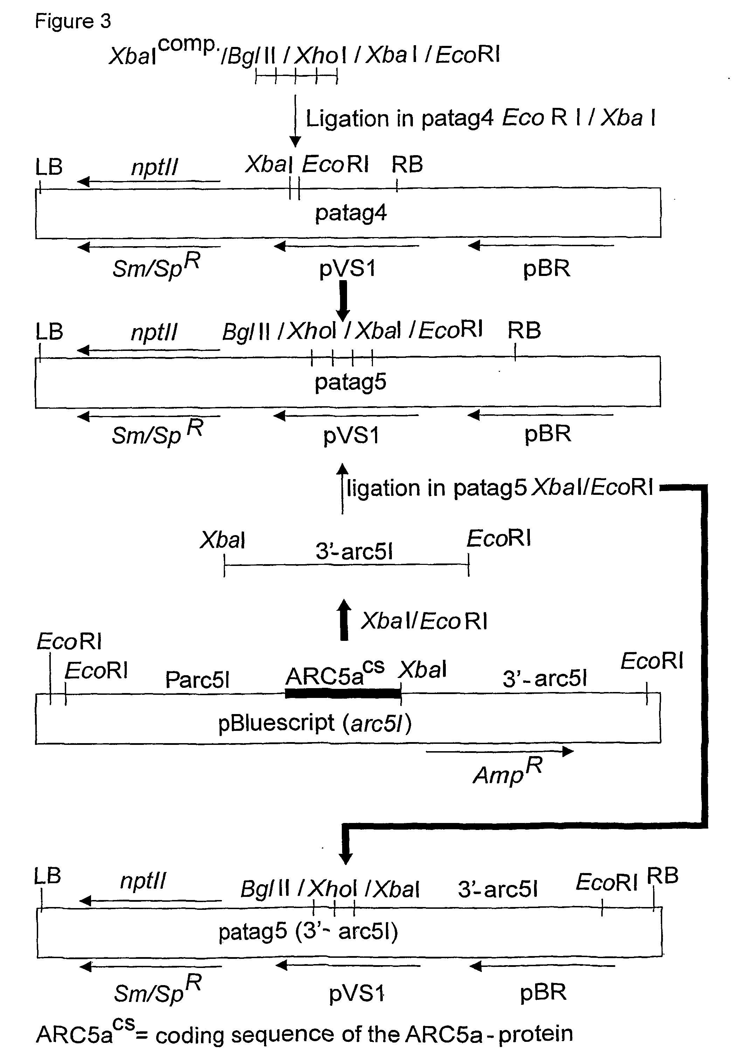

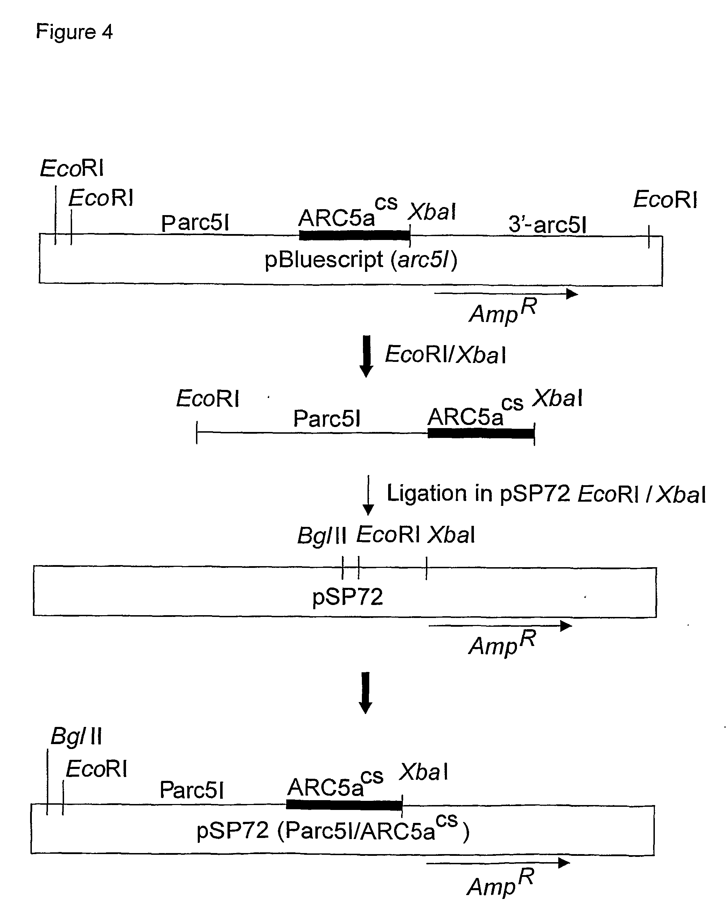

Four T-DNA vectors were constructed to evaluate and compare scFv production under control of the *arc5l* expression signals (vectors pParc5l-G4 and pParc5l-Ω-G4), the 35S promoter of the cauliflower mosaic virus (vector pP35S-G4), and the promoter of the *β-phaseolin* gene of *Phaseolus vulgaris* (vector pPβ-phas-G4) (figure 1). A first step in the cloning procedure consisted of the construction of three pilot vectors: pBluescript (2S2-G4) (figure 2), patag5 (3'-arc5l) (figure 3) en pSP72 (Parc5l/ARC5a^{cs}) (figure 4).

### Construction of pBluescript (2S2-G4) (figure 2):

The coding sequence of the scFv fragment G4, fused at its 3'-end to the coding sequence of the c-*myc* tag (Evan *et al*., 1985) and the ER-retention signal KDEL (Denecke *et al*., 1992), was cut from the T-DNA vector pG4ER (De Jaeger et al., 1999) by the restriction sites *Nco*I and *Xba*l*.* Besides, an oligonucleotide was made that encodes the signal sequence of the seed storage protein 2S2 from *Arabidopsis* *thaliana* (Krebbers *et al*., 1988). This oligonucleotide was flanked at its 3'-end by the 'sticky' end of the restriction site *Nco*l and was flanked at its 5'-end by a 'sticky' end that complements the restriction site *Xba*l but destroys it after ligation, followed by the restriction sites *Eco*RI, *Bgl*II en *Hind*III. The G4 fragment and the oligonucleotide were cloned together in the vector pBluescriptKS (Stratagene, La Jolla, CA), after cutting with *Hind*III en *Xba*l. The sequence of the insert was checked and a clone containing a correct insert was selected for further cloning steps. As such, we obtained the pilot vector pBluescript (2S2-G4), which contained the G4-encoding sequence, preceded by a series of unique restriction sites that could be used for the insertion of the different promoters in combination with a mRNA-leader sequence.

### Construction of patag5 (3'-arc5l) (figure 3):

An oligonucleotide was made to insert a few unique restriction sites in the T-DNA-vector patag4 (Goossens *et al*., 1999). The oligonucleotide contained the following restriction sites from its 5'-end to its 3'-end: the 5'-'sticky' end of *Eco*RI, the restriction sites *Xba*l, *Xho*l, and *Bgl*II and a 3'-'sticky' end that complements *Xba*l but destroys it after ligation. The oligonucleotide was ligated in patag4, after cutting with *Eco*RI en *Xba*l*.* This resulted in the vector patag5. The sequence of the inserted oligonucleotide was checked and a clone with correct insert was selected for further cloning steps. From the vector pBluescript (*arc5l*) (Goossens *et al.,* 1995), which contains the genomic sequence of the *arc5l*-gene, we cut the 3'-expression signals of *arc5l* (3'-arc5l) by using *Xba*l en *Eco*RI. The 3'-arc5l-fragment was ligated in patag5, after cutting with *Xba*l en *Eco*RI. This resulted in the T-DNA-vector patag5 (3'-arc5I).

### Construction of pSP72 (Parc5l/ARC5a^{cs}) (figure 4):

The *arc5l* promoter and the coding sequence of the ARC5a-protein (ARC5a^{cs}) were cut from pBluescript (*arc5l*). This fragment was ligated in the cloning vector pSP72 (Promega, Madison, WI) after cutting with *Eco*RI en *Xba*l*.* This resulted in the vector pSP72 (Parc5l/ARC5a^{cs}), containing the *arc5l*-promoter preceded by the restriction sites *Eco*RI and *Bgl*II.

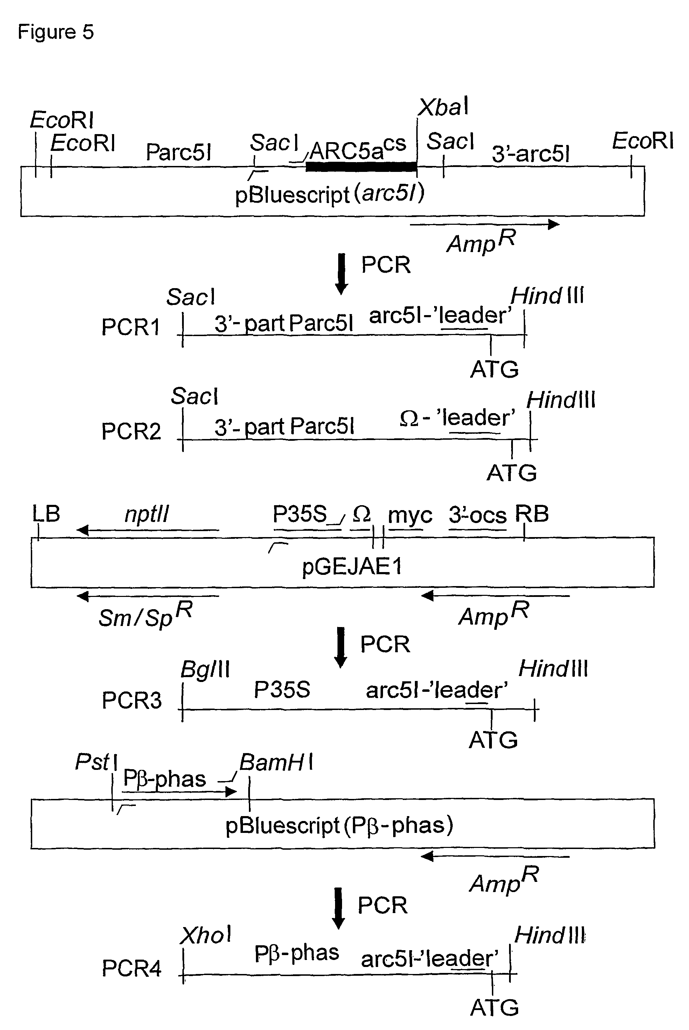

Besides these three pilot-vectors, four DNA fragments were amplified by PCR. These fragments were called PCR1, PCR2, PCR3 en PCR4 (figure 5). Fragments PCR1 en PCR2 were amplified from the 3'-end of the *arc5l*-promoter (Parc5l) in pBluescript (*arc5l*). PCR1 contained the 3'-end of Parc5l followed by the *arc5l*-'leader' and the 5'-end of the 2S2 signal sequence. PCR2 contained the same 3'-end of Parc5l, but followed by the Ω-'leader' and the 5'-end of the 2S2 signal sequence. Both fragments were flanked by the restriction sites *Sac*I en *Hind*III. PCR3 was obtained by amplifying the 35S promoter of the cauliflower mosaic virus from the vector pGEJAE1 (De Jaeger *et al*., 1999). At the 3'-end of the 35S promoter, we built in the *arc5I*-'leader', followed by the 5'-end of the 2S2 signal sequence. PCR3 was flanked by the restriction sites *Bgl*II and *Hind*III. PCR4 contained the promoter of the β-*phaseolin* gene of *Phaseolus vulgaris,* amplified from the vector pBluescript (Pβ-phas) (van der Geest *et al*., 1994). At the 3'-end of PCR4, we built in the *arc5l*-'leader' followed by the 5'-end of the 2S2-signal sequence. This fragment was flanked by the restriction sites *Xho*l and *Hind*III. The pilot vectors and the four PCR fragments were used to clone the four T-DNA vectors from figure 1.

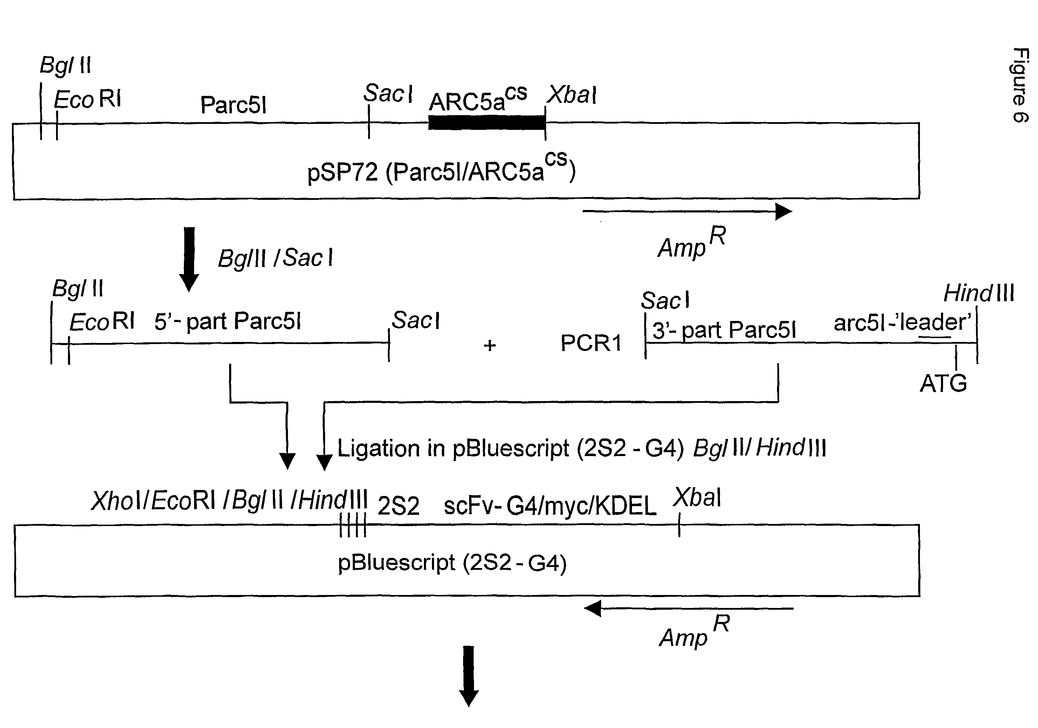

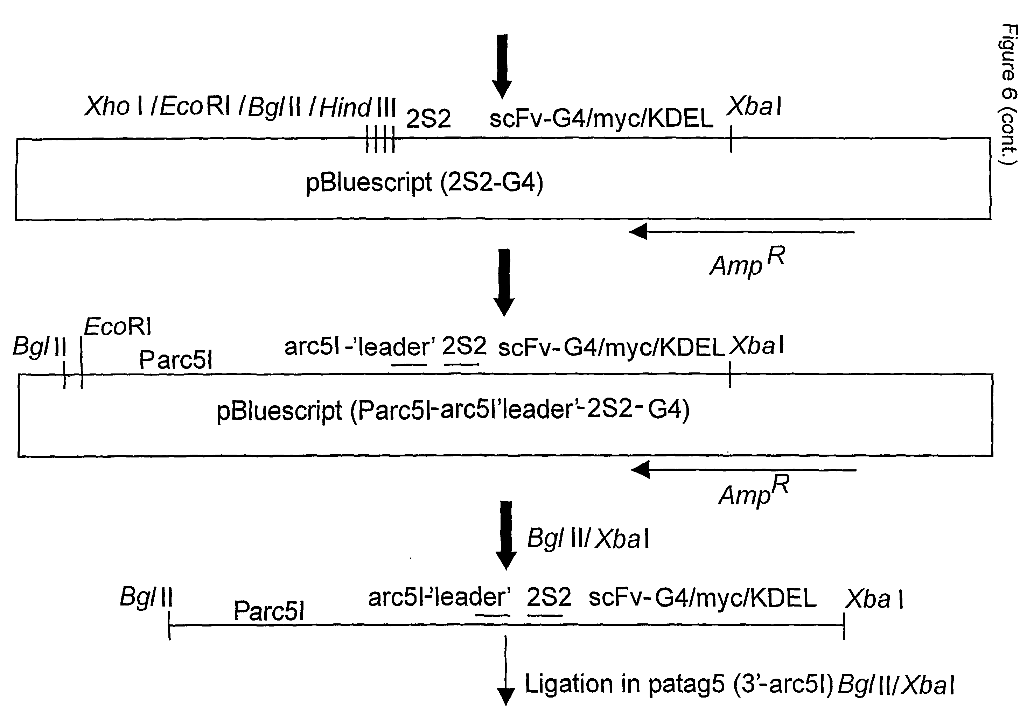

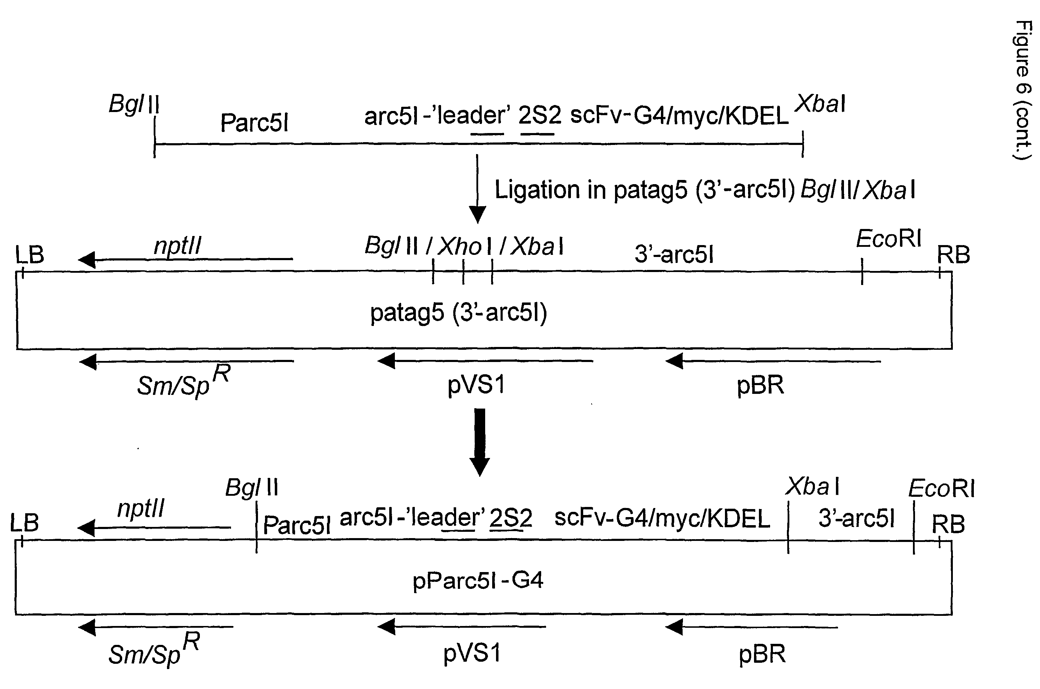

### Construction of pParc5l-G4 (figure 6):

First, the 5'-end of the *arc5I* promoter was cut from the vector pSP72 (Parc5l/ARC5a^{cs}) by using *Bgl*II and *Sac*I enzymes. This promoter fragment, together with the PCR1 fragment, was ligated in de vector pBluescript (2S2-G4), after restriction digest with

*Bgl*II en *Hind*III. This resulted in the vector pBluescript (Parc5l-arc5l'leader'-2S2-G4). The sequence of the inserted PCR1 fragment was checked and a clone with correct insert was selected for further cloning steps. Finally, the *arc5I*-promoter with the *arc5I*-'leader' and the G4-coding sequence was cut from the former construct by the restriction sites *Bgl*II en *Xba*l and ligated in the vector patag5(3'-arc5l), after digestion with *Bgl*II en *Xbal.* This resulted in the T-DNA vector pParc5l-G4. This plasmid, transformed in *E. coli* MC1061 is deposited at BCCM under deposit number LMBP 4128. The plasmid contains the full *arc5I*-promoter and the full 3'end of *arc5l*, as used in the expression cassettes comprising the *arc5l*-promoter and the 3'end of *arc5l*.

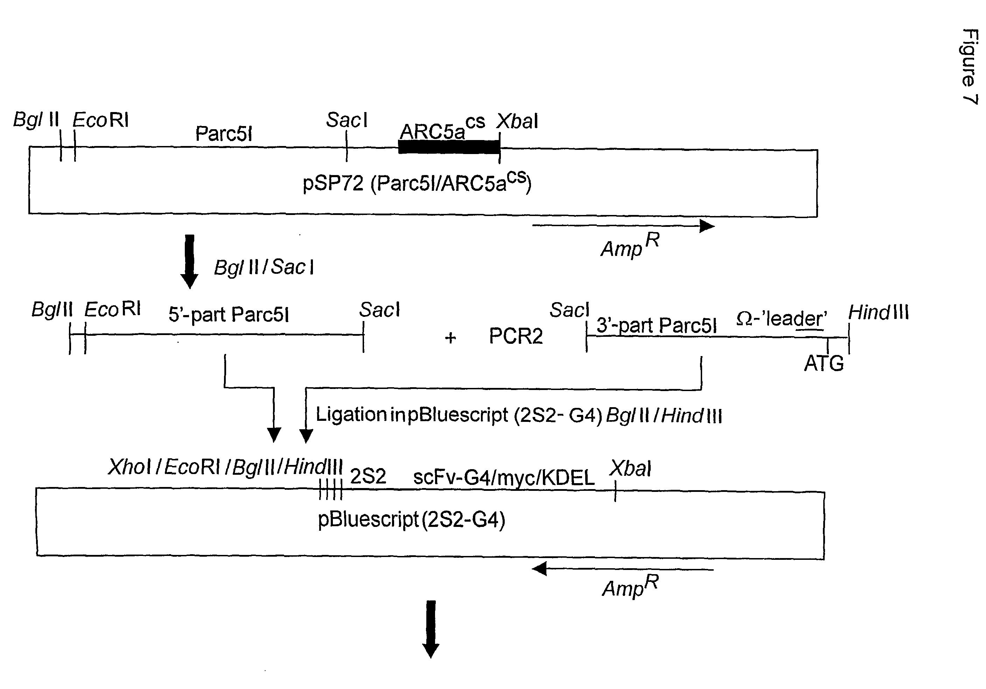

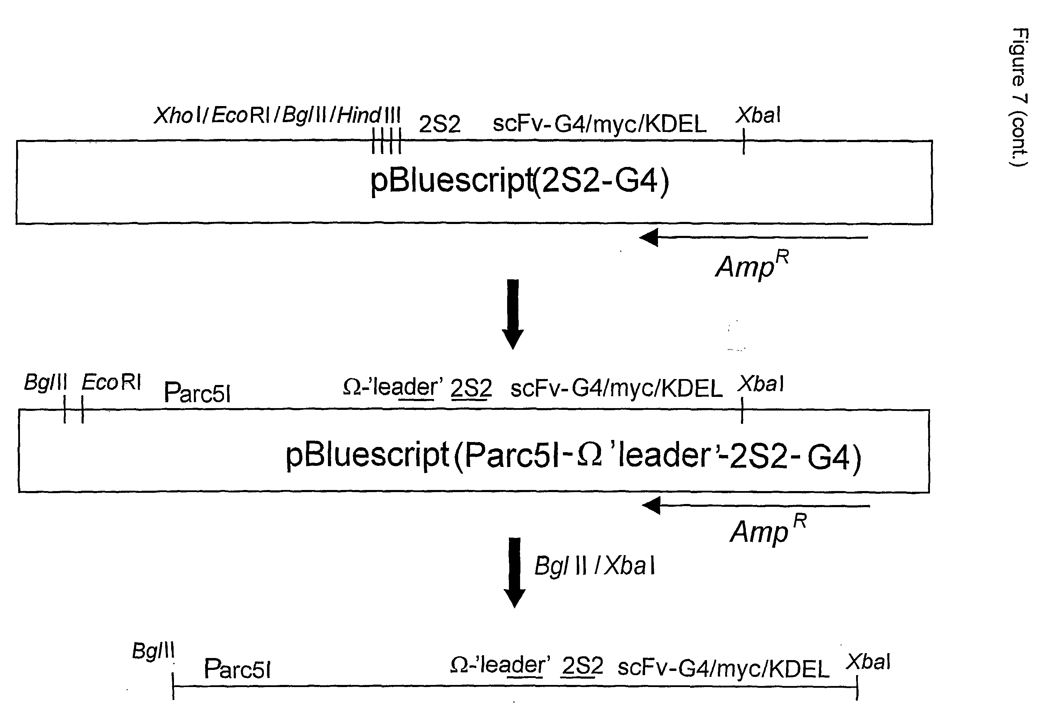

### Construction of pParc5l-Ω-G4 (figure 7):

First, the 5'-end of the *arc5l* promoter was cut from the vector pSP72 (Parc5l/ARC5a^{cs}) by restriction digest with *Bgl*II and *Sac*I. This promoter fragment, together with the PCR2 fragment, was ligated in de vector pBluescript (2S2-G4), after restriction digest with *Bgl*II en *Hind*lll*.* This resulted in the vector pBluescript (Parc5l-Ω'leader'-2S2-G4). The sequence of the inserted PCR2 fragment was checked and a clone with correct insert was selected for further cloning steps. Finally, the *arc5l*-promoter with the Ω-'leader' and the G4-coding sequence was cut from the former construct by the restriction sites *Bgl*II en *Xba*l and ligated in the vector patag5 (3'-arc5l), after digestion with *Bgl*II en *Xba*l*.* This resulted in the T-DNA vector pParc5l-Ω-G4.

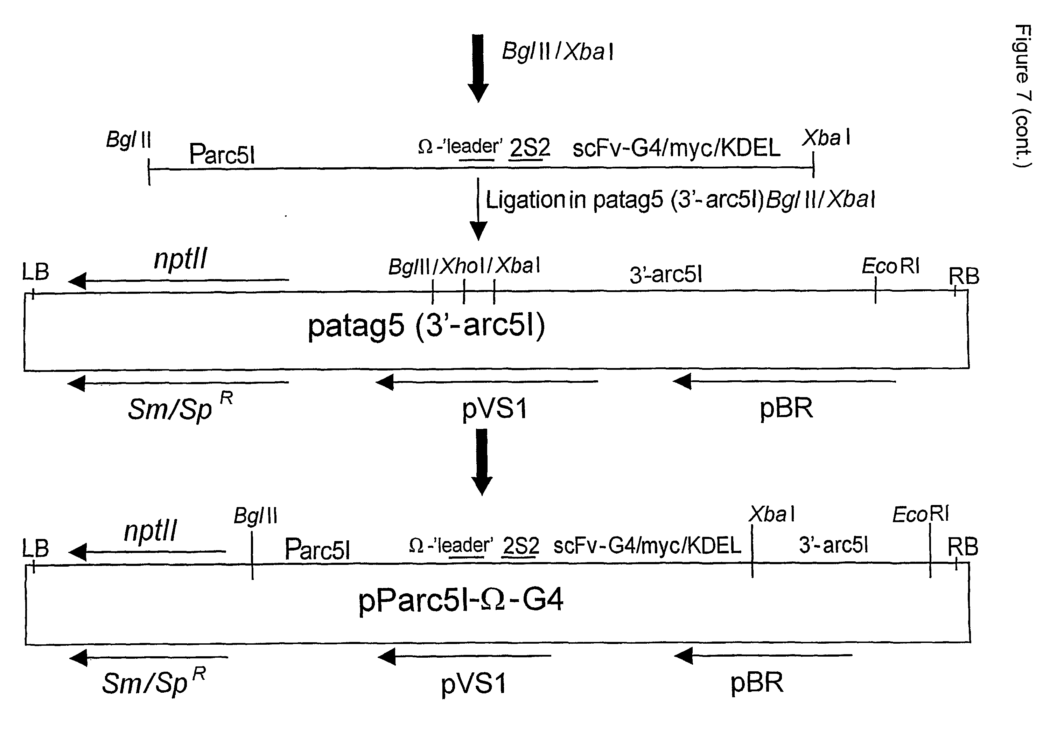

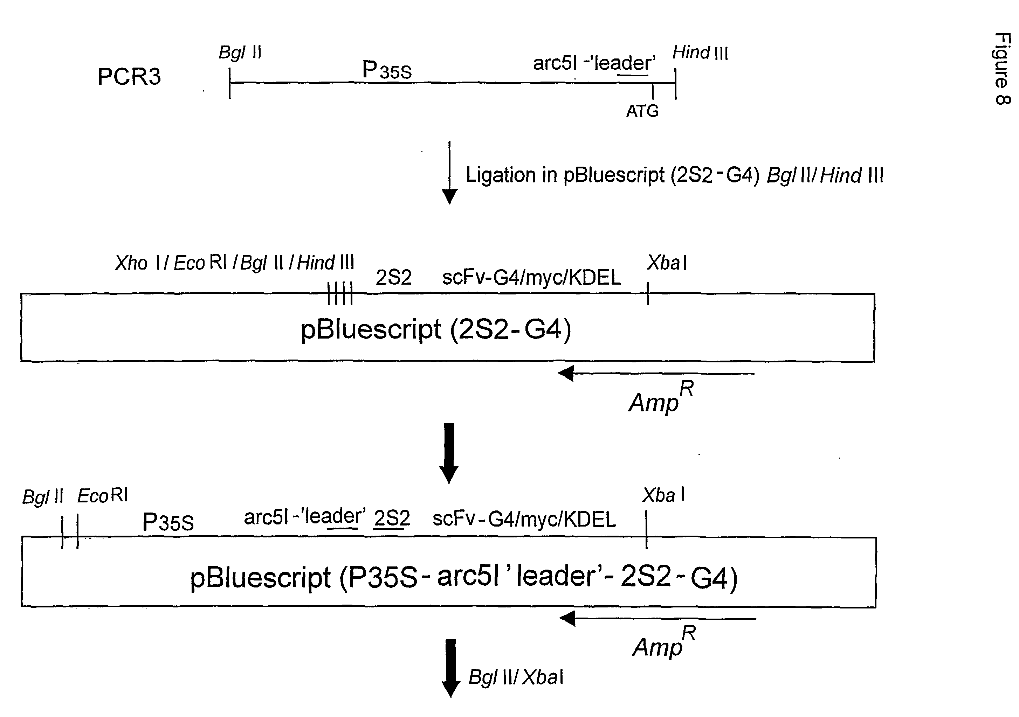

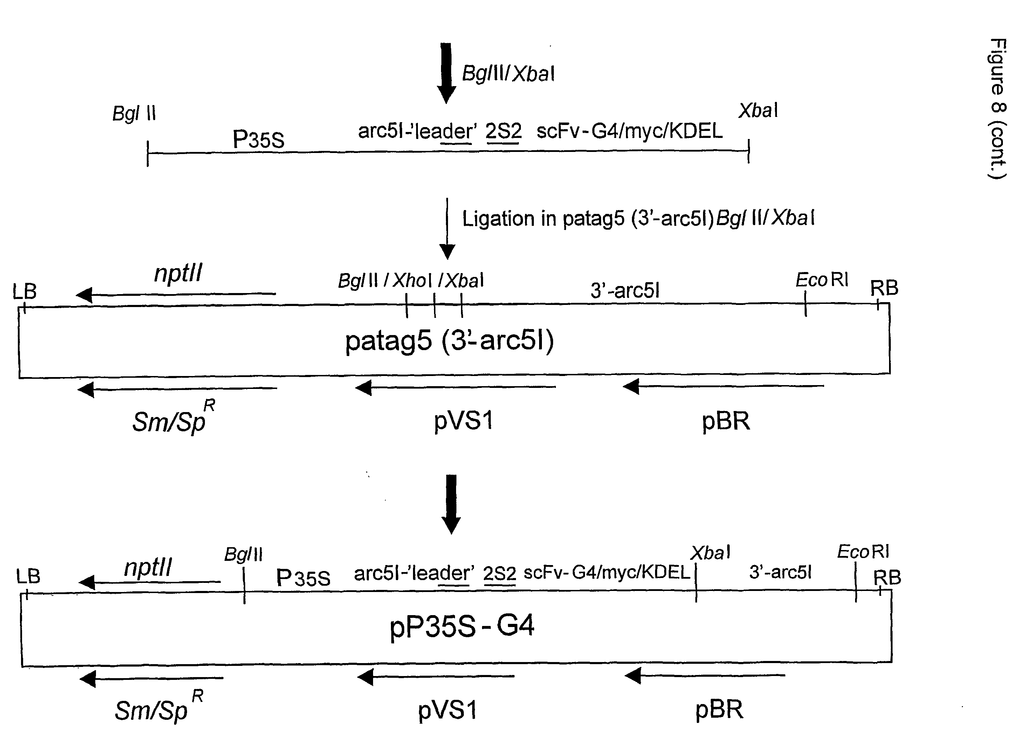

### Construction of pP35S-G4 (figure 8):

The PCR3 fragment was ligated in the vector pBluescript (2S2-G4), after restriction digest with *Bgl*II and *Hind*III. This resulted in the vector pBluescript (P35S-arc5l'leader'-2S2-G4). The sequence of the inserted PCR3 fragment was checked and a clone with correct insert was selected for further cloning steps. Finally, the 35S-promoter with the arc5l-'leader' and the G4-coding sequence was cut from the former construct by the restriction sites *Bgl*II en *Xba*l and ligated in the vector patag5 (3'-arc5l), after digestion with *Bgl*II en *Xba*l*.* This resulted in the T-DNA vector pP35S-G4.

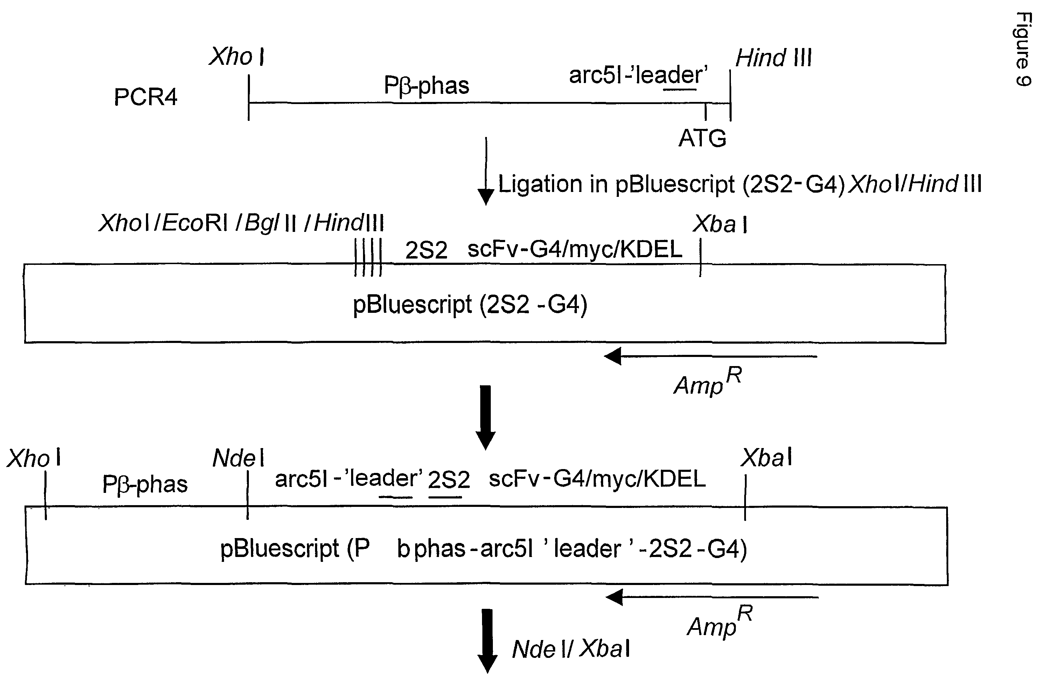

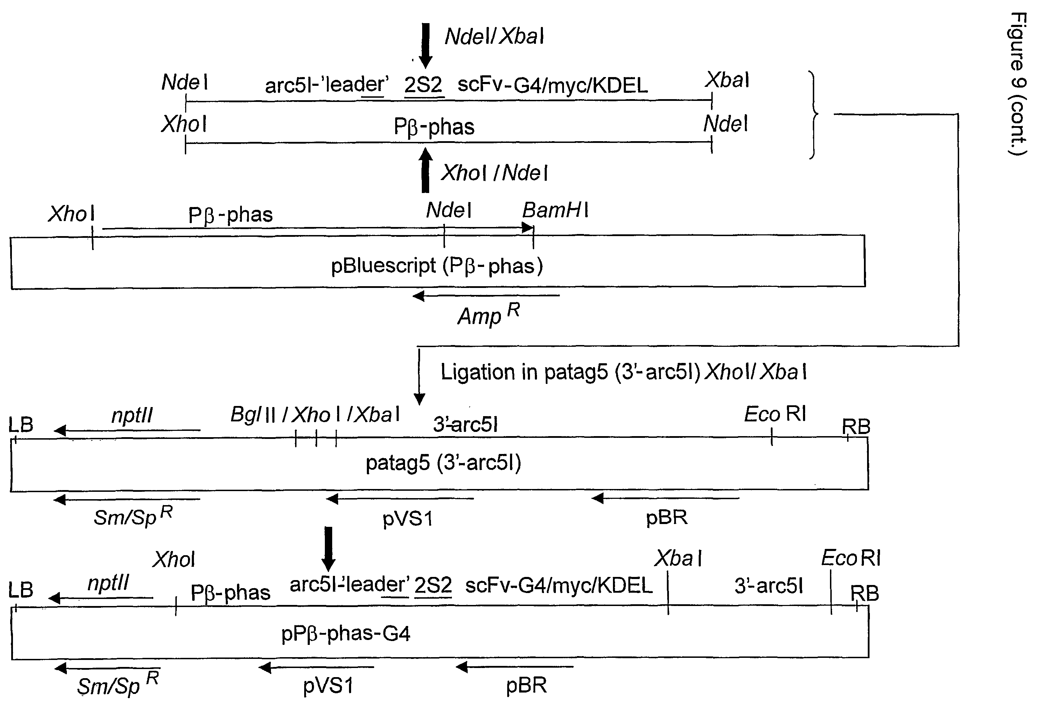

### Construction of pPβ-phas-G4 (figure 9):

The PCR4 fragment was ligated in the vector pBluescript (2S2-G4), after restriction digest with *Xho*l en *Hind*III. This resulted in the vector pBluescript (Pβphas-arc5l'leader'-2S2-G4). After checking the DNA-sequence of the inserted PCR4-fragment, we found in the *β-phaseolin* promoter a few basepairs that differed from the original sequence (Bustos *et al*, 1991;Genbank accession number J01263). However, the 3'-end of the cloned promoter sequence, starting from the Ndel-site, was completely the same as the given sequence. Therefore, this piece, together with the coding sequence of scFv G4, was cut from the vector pBluescript (Pβphas-arc5l'leader'-2S2-G4) by using the restriction sites *Nde*I en *Xba*l*.* Besides, the 5'-end of the β-*phaseoline-*promoter was cut from pBluescript (Pβ-phas) by the restriction sites *Xho*l en *Nde*l*.* Both DNA-fragments were ligated in patag5 (3'-arc5l), after restriction digest with *Xho*l en *Xba*l*.* This resulted in the final vector pPβ-phas-G4. Again we checked the sequence of the *β-phaseoline*-promoter and the same differences were found with the original sequence. The sequence between the *Xh*ol site and the *Nde*l site, as used in the construct is depicted in SEQ ID N° 5.

The four T-DNA vectors were purified from *Escherichia coli* and electroporated in *Agrobacterium tumefaciens* C58C1Rif^{R} (pMP90). After colony purification, plasmids were purified from *Agrobacterium* and checked. The *Agrobacterium-*strains were used in *Arabidopsis* transformation.

### Example 2: Transformation of Arabidopsis thaliana and regeneration of transgenic plants

*Arabidopsis thaliana* (*Columbia genotype O*) was transformed by root-transformation (Valvekens et al., 1988) with the constructs pParc5l-G4, pParc5l-Ω-G4, pP35S-G4 en pPβ-phas-G4. After selection of transformed calli on kanamycin-selective medium, 150 calli were transferred to shoot inducing medium. Finally, shoots were transferred to root inducing medium. After root formation plants were transfered to the greenhouse and seeds were collected from the following numbers of transgenic *Arabidopsis* plants: 36 for pParc5l-G4, 4 for pP35S-G4, 18 for pPβ-phas-G4, and 13 for pParc5l-Ω-G4.

In parallel, the same constructs were used for *Arabidopsis* transformation by 'floral dip' (Clough & Bent, 1998). Transformed T1-plants were selected on kanamycin-containing selective medium, transferred to the greenhouse, and seeds were collected.

### Example 3: scFv accumulation in transgenic Arabidopsis seeds

### Seed extraction and protein quantification

Crude seed protein extracts were obtained following a modification of the extraction protocol of van der Klei *et al*. (1995) (Goossens *et al*., 1999). Ground seeds were extracted twice with hexane to remove lipids. The residue was lyophilized and subsequently extracted twice with 50 mM Tris/HCl, 200 mM NaCl, 5 mM EDTA, 0,1% Tween 20, pH 8 (Fiedler *et al*., 1997) for 15 min at room temperature under continuous shaking. To prevent protein degradation, a protease inhibitor mix (2x CØmplete™, Roche Molecular Biochemicals, Germany) was added to the extraction buffer. The pellet was removed by centrifugation at 20000x*g* and supernatants were pooled. Total protein quantity in the crude extracts was determined by the Lowry method using the DC Protein Assay (BioRad, Hercules, USA) with BSA as a standard (Table 1). The reliability of *Arabidopsis* seed protein quantification by this method was proven by Goossens *et al*., 1999.

**Table 1:**

| Total protein concentration in transgenic seed extracts (500 microliter) from 10 mg transgenic *Arabidopsis* seeds | | | | |
|---|---|---|---|---|
| **A 1** | **A 105** | **A 140** | **A 143** | **A 165A** |
| 3,665 µg/µl | 3,395 µg/µl | 3,042 µg/µl | 3,708 µg/µl | 3,675 µg/µl |

| **P 3A** | **P 5** | **P 15** | **P 22A** | **P 102B** |
|---|---|---|---|---|
| 2,852 µg/µl | 4,028 µg/µl | 3,623 µg/µl | 3,151 µg/µl | 3,339 µg/µl |

| **Ω 7A** | **Ω 33** | **Ω 65C** | **Ω 98A** | **Ω 130** |
|---|---|---|---|---|
| 3,873 µg/µl | 3,527 µg/µl | 3,184 µg/µl | 3,754 µg/µl | 3,517 µg/µl |

| **35S 93** | **35S 101** | **35S 116** | **35S 131A** | **Col O** |
|---|---|---|---|---|
| 3,945 µg/µl | 3,837 µg/µl | 3,879 µg/µl | 3,906 µg/µl | 3,215 µg/µl |
| Col O = non-transformed *Columbia genotype O* used as a control | | | | |
| A = pParc5l-G4 transformed *Arabidopsis* line | | | | |
| P = pPβ-phas-G4 transformed *Arabidopsis* line | | | | |
| 35S = pP35S-G4 transformed *Arabidopsis* line | | | | |
| Ω = pParc5I-Ω-G4 transformed *Arabidopsis* line | | | | |

### 3.2. Quantification of scFv-G4 accumulation

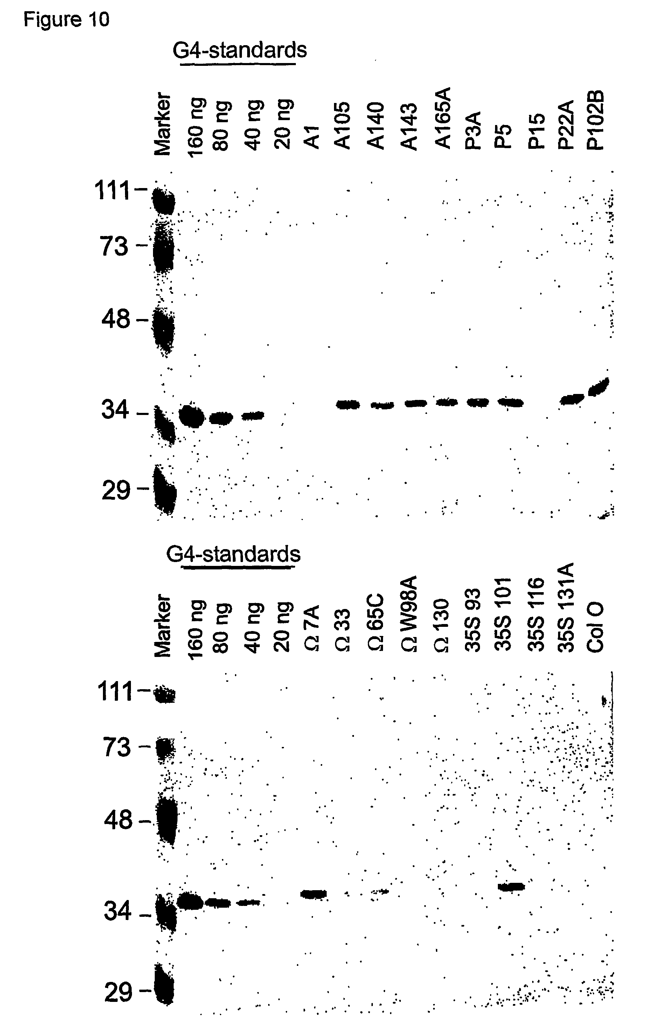

Proteins were separated on SDS/PAGE and accumulation levels of scFv-G4 proteins were determined by quantitative Western blot analysis using the anti-c-*myc* monoclonal antibody 9E10 (Evan *et al*., 1995) followed by anti-mouse IgG coupled to alkaline phosphatase (Sigma, St Louis, MO, USA), according to De Jaeger *et al.,* 1999 (figure 10). Different amounts of scFv-G4 proteins purified from *Escherichia coli* (De Jaeger *et al*., 1999) were used as standards. The constructs pParc5l-G4, pParc5l-Ω-G4, and pPβ-phas-G4 give very high scFv-G4 accumulation levels in *Arabidospsis* seeds, in the range of 10% of total soluble seed protein. These are the highest levels ever reported for scFv proteins produced in plants. Moreover, lines with such high levels were easily found, as only 5 lines were screened for each construct, which is an indication that the expression cassettes are not very sensitive to silencing.

**Table 2:**

| ScFv-G4 accumulation levels in transgenic *Arabidopsis* seeds. | | | | | | | |
|---|---|---|---|---|---|---|---|
| **PParc5I-G4** | | **pPβ-phas-G4** | | **pParc5I-Ω-G4** | | **pP35S-G4** | |
| Line | G4-level | Line | G4-level | Line | G4-level | Line | G4-level |
| | (*) | | (*) | | (*) | | (*) |
| A 1 | < 4% | P 3A | 10% | Ω 7A | 12% | 35S93 | < 0,8% |
| A 105 | 10% | P 5 | 10% | Ω 33 | < 4% | 35S | 3% |
| | | | | | | 101 | |
| A 140 | 6% | P 15 | < 4% | Ω 65C | 6% | 35S | < 0,8% |
| | | | | | | 116 | |
| A 143 | 8% | P 22A | 12% | Ω 98A | < 4% | 35S | < 0,8% |
| | | | | | | 131A | |
| A 165A | 8% | P 102B | 12% | Ω 130 | < 4% | | |

| | | | | | | | |
|---|---|---|---|---|---|---|---|
| (*) accumulation level as % of total soluble protein content in transgenic seeds | | | | | | | |

### Example 4: Transformation of Arabidopsis thaliana by 'floral dip' and regeneration of transgenic plants

*Arabidopsis thaliana* (Columbia genotype O) was transformed by 'floral dip' (Clough & Bent, 1998) with the constructs pParc5l-G4, pParc5l-Ω-G4, pP35S-G4, and pPβ-phas-G4. Transformed T1-plants were selected on kanamycin-containing selective medium, transferred to the greenhouse, and T2-segregating seed stocks were collected.

### ScFv accumulation in transgenic T2-segregating seed stocks

### Seed extraction and protein quantification

Seed extraction and protein quantification was determined as described for Example 3.

### Quantification of scFv-G4 accumulation

Proteins were separated on SDS/PAGE and accumulation levels of scFv-G4 proteins were determined by quantitative Western blot analysis using the anti-c-myc monoclonal antibody 9E10 (Evan et al., 1995) followed by anti-mouse IgG coupled to alkaline phosphatase (Sigma, St Louis, MO, USA), according to De Jaeger *et al*., 1999 (figure 11). Different amounts of scFv-G4KDEL proteins purified from *Escherichia coli* were used as standards. Most seed stocks were at least two times analysed. The constructs pParc5l-G4 and pPβ-phas-G4 give very high scFv-G4 accumulation levels in *Arabidopsis* seeds, in the range of 5-10% and 10%-20% of total soluble seed protein, respectively (tabel 3). Use of the *arc5l*-untranslated leader in pParc5l-G4 or the TMV(omega)-leader in pParc5l-Ω-G4 give similar accumulation levels (tabel 3), showing that both leaders allow efficient translation start in seeds. Besides, intertransgenic variation is low for all four constructs, which is an indication that the expression cassettes are not very sensitive to silencing. Seed extracts of four pPβ-phas-G4 plant lines with highest G4-accumulation were further analysed by SDS/PAGE and Coomassie-blue staining (figure 12). A clear scFv-protein band could be identified at the expected size, which was absent in the untransformed control line. By using the Imagemaster VDS software (Pharmacia, Uppsala, Sweden), the scFv-percentage of total soluble seed protein was measured in each lane. Similar scFv-accumulation levels were obtained for those lines as with the quantitative Western blot analysis.

**Tabel 3:**

| ScFv-G4 accumulation levels in transgenic *Arabidopsis* segregating T2- seed stocks. 20 independent seed stocks were analysed, scFv levels were ranked from highest to lowest. | | | |
|---|---|---|---|
| pParc5I-G4 (*) | pPβ-phas-G4 (*) | pParc5I-Ω-G4 (*) | pP35S-G4 (*) |
| 8.0 ± 1.4 | 20.0 ± 0.0 | 5.8 ± 0.4 | 1.1 ± 0.1 |
| 7.8 ± 0.4 | 19.0 ± 1.4 | 4.9 ± 0.2 | 1.1 ± 0.1 |
| 6.7 ± 0.1 | 12.0 ± 0.0 | 4.5 ± 0.7 | 1.1 ± 0.1 |
| 6.4 ± 0.5 | 11.3 ± 1.8 | 4.0 ± 0.0 | 1.0 ± 0.0 |
| 5.3 ± 1.8 | 7.2 ± 0.2 | 3.9 ± 0.2 | 1.0 ± 0.0 |
| 4.7 ± 0.5 | 5.4 ± 0.9 | 3.9 ± 0.2 | 0.8 ± 0.1 |
| 4.5 ± 0.7 | 5.0 ± 0.0 | 3.5 ± 0.2 | 0.7 ± 0.1 |
| 3.9 ± 0.2 | 4.5 ± 0.7 | 3.2 ± 0.2 | 0.7 ± 0.1 |
| 3.8 ± 0.4 | 4.4 ± 0.5 | 3.1 ± 0.6 | 0.7 ± 0.1 |
| 3.7 ± 0.5 | 4.0 ± 1.0 | 3.0 ± 0.0 | 0.7 ± 0.0 |
| 2.4 ± 0.5 | 3.9 ± 0.2 | 3.0 ± 0.0 | 0.7 ± 0.1 |
| 1.7 ± 0.5 | 3.8 ± 0.3 | 2.7 ± 0.4 | 0.7 ± 0.1 |
| 1.3 ± 0.4 | 1.8 ± 0.4 | 1.6 ± 0.4 | 0.6 ± 0.0 |
| 0.9 ± 0.2 | 1.8 ± 0.3 | 1.5 | 0.5 ± 0.1 |
| 0.8 ± 0.0 | 1.6 | 1.4 ± 0.1 | 0.5 ± 0.0 |
| 0.5 ± 0.1 | 1.3 | 0.7 ± 0.0 | 0.4 ± 0.0 |
| 0.5 ± 0.1 | 1.2 | 0.6 ± 0.1 | 0.3 ± 0.0 |
| 0.4 ± 0.1 | 0.8 | 0.3 | 0.2 ± 0.1 |
| <0.3 | <0.4 | 0.2 | 0.1 |
| <0.3 | n.d. | 0.1 | n.d. |

| | | | |
|---|---|---|---|
| (*) scFv accumulation level as % of total soluble protein content in transgenic seeds, with standard deviation. n.d. = not detectable | | | |

### ScFv accumulation in transgenic T3-homozygous seed stocks

For each construct, 10 T2-seed stocks containing highest G4-levels were genetically screened by segregation analysis. 72 seeds from each seed stock were germinated on kanamycin-containing selective medium and by statistical analysis we identified plant lines containing a single T-DNA locus. For the constructs pParc5l-G4 and pPβ-phas-G4, 4 lines containing a single T-DNA locus were chosen to grow and select homozygous seed stocks. 10 T2-plants per line were grown, T3-seeds were collected and homozygous T3-seed stocks were selected by growing plants on kanamycin-containing selective medium. For one of the four lines containing construct pParc5l-G4, no homozygous seed stocks were found. G4-accumulation was measured by quantitative Western blot in T3-segregating and T3-homozygous seed stocks.

Most pParc5l-G4 and all pPβ-phas-G4 homozygous seed stocks gave higher G4-levels than the corresponding T3-segregating stocks (tabel 4). For both constructs pParc5l-G4 and pPβ-phas-G4, homozygous seed stocks were obtained containing more than 10% G4 of total soluble seed protein. Homozygous seed stocks, transformed with pPβ-phas-G4, contained extraordinary high G4 levels, up to 36.5% of TSP in seeds. This is the highest heterologous protein level ever reported for plants.

**Tabel 4:**

| ScFv-G4 accumulation levels in transgenic *Arabidopsis* segregating and homozygous T3-seed stocks. | | | |
|---|---|---|---|
| **Plant line** | **T2-segregating seed stocks (*)** | **T3-segregating seed stocks (*)** | **T3-homozygous seed stocks (*)** |
| A1 | 8.0 ± 1.4 | 8.2 ± 1.0 | 4.4 ± 0.8 |
| | | 6.7 ± 0.7 | 12.5 ± 1.9 |
| A15 | 4.7 ± 0.5 | 5.6 ± 1.0 | 6.4 ± 1.4 |
| | | 4.7 ± 0.0 | 7.7 ± 1.4 |
| A16 | 4.5 ± 0.7 | 3.8 ± 0.7 | 6.0 ± 1.0 |
| | | 5.1 ± 1.4 | 3.5 ± 0.4 |
| F3 | 4.5 ± 0.7 | 5.8 | 18.0 |
| F24 | 5.0 ± 0.0 | 7.0 ± 1.4 | 13.5 ± 2.1 |
| | | 4.9 ± 0.2 | 15.0 ± 1.4 |
| F28 | 11.3 ± 1.8 | 13.0 ± 1.4 | 21.0 ± 4.2 |
| | | 10.5 ± 0.7 | 18.5 ± 0.7 |
| F38 | 19.0 ± 1.4 | 17.5 ± 0.7 | 36.5 ± 3.4 |
| | | 17.5 ± 3.5 | |

| | | | |
|---|---|---|---|
| (*) scFv accumulation level as % of total soluble protein content in transgenic seeds, with standard deviation. n.d. = not detectable. For most lines more than one T3-segregating and homozygous seed stocks were analysed. A1, A15, and A16 are plant lines transformed with construct pParc5l-G4. Lines F3, F24, F28, and F38 were transformed with pPβ-phas-G4. | | | |

### Analysis of scFv-quality in seed extracts

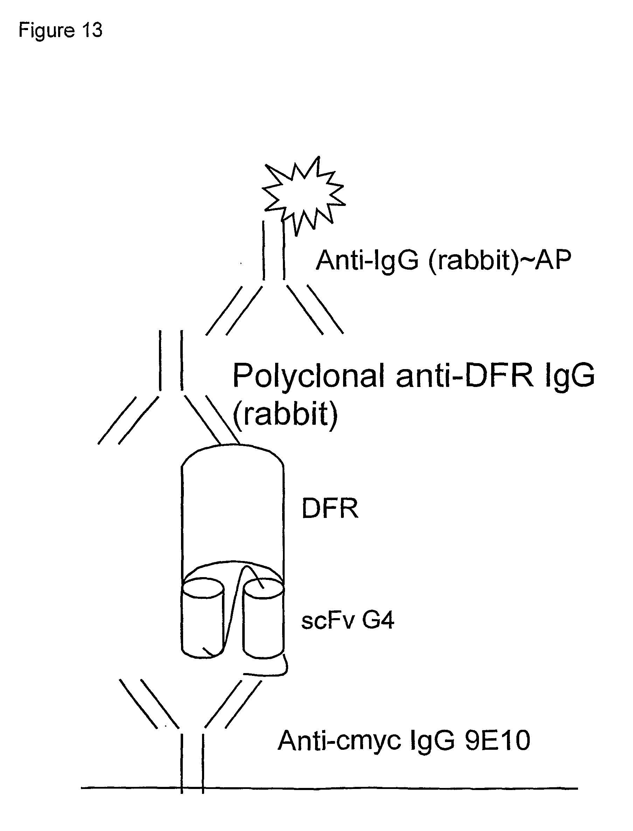

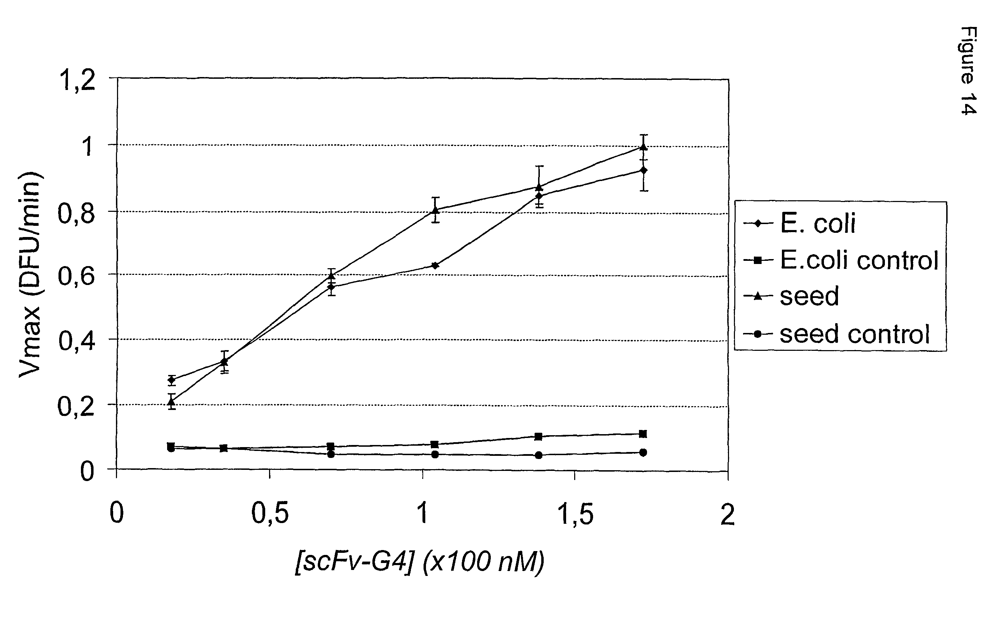

Antigen-binding activity of seed extracted G4-proteins was measured and compared with *E. coli*-extracted G4 by ELISA. We used seed extract of the 'phas'-seed stock containing 36.5% G4 (tabel 4). Different amounts of scFv were incubated with the antigen dihydroflavonole-4-reductase in excess and bound antigen was measured by sandwich-ELISA (figure 13). ELISA-signal curves were set up for both the bacterial and plant scFv and compared. The curves overlap each other (figure 14), indicating that the plant-produced scFv has similar antigen-binding activity as the bacterial scFv.

### Example 5: Transformation of Phaseolus acutifolius and scFv accumulation in transgenic segregating bean seeds

*Phaseolus acutifolius* TB1 was transformed with pParc5l-G4bis (figure 16). pParc5l-G4bis contains the same T-DNA as pParc5l-G4, except that it contains an additional P35S-GUS-construct for segregation analysis of transgenic plants.

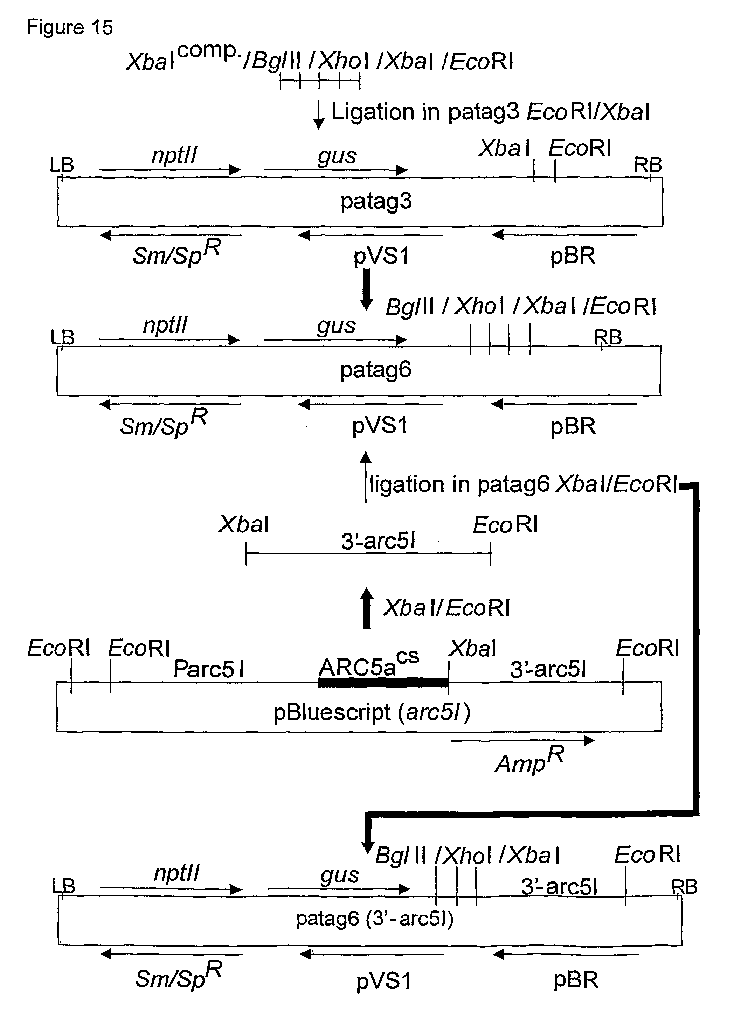

For the construction of this T-DNA vector, we first made the pilot construct patag6(3'-arc5l) (figure 15) according to the cloning step procedure for patag5(3'-arc5l) (figure3). An oligonucleotide was made to insert a few unique restriction sites in the T-DNA-vector patag3 (Goossens *et al*., 1999). The oligonucleotide contained the following restriction sites from its 5'-end to its 3'-end: the 5'-'sticky' end of *Eco*RI, the restriction sites *Xba*I, *Xho*l*,* and *Bgl*II and a 3'-'sticky' end that complements *Xba*l but destroys it after ligation. The oligonucleotide was ligated in patag3, after cutting with *Eco*RI en *Xba*l*.* This resulted in the vector patag6. The sequence of the inserted oligonucleotide was checked and a clone with correct insert was selected for further cloning steps. From the vector pBluescript (*arc5l*) (Goossens *et al*., 1995), which contains the genomic sequence of the *arc5l*-gene, we cut the 3'-expression signals of *arc5l* (3'-arc5l) by using *Xba*l en *Eco*RI. The 3'-arc5l-fragment was ligated in patag6, after cutting with *Xba*I en *Eco*RI. This resulted in the vector patag6 (3'-*arc*5l). This pilot construct was used to make pParc5l-G4bis (figure 16). The *arc5l*-'leader' and the G4-coding sequence was cut from the vector pBluescript (Parc5l-arc5l'leader'-2S2-G4) (figure 6) by the restriction sites *Bgl*II en *Xba*l and ligated in the vector patag6 (3'-arc5l), after digestion with *Bgl*II en *Xba*I. This resulted in the T-DNA vector pParc5l-G4bis.

Three transgenic plants were obtained by using the protocol of Dillen *et al*. (1997). As the three plants were regenerated from the same callus, it was expected that they were clones from the same transformation event. Seeds were collected and protein extracts were made from separate seeds in the same buffer as the *Arabidopsis* seed extraction, and according to Goossens *et al*. (1999). Total soluble protein concentration was measured spectrophotometrically at 280nm and G4 accumulation was determined by quantitative Western blot.

G4 was detected as a single protein band (figure 17) in, on average, 3 of 4 seeds for all three segregating seed stocks. Therefore, these transformants most probably contain a single T-DNA-locus. All analysed G4-accumulating seeds contained 2-2,5% G4 of total soluble protein or 2-2,5 milligram scFv per gram fresh weight seed. Sofar, only one paper reported scFv production in leguminous species. Perrin *et al*. (2000) obtained 9 microgram scFv per gram fresh weight in pea seeds with the *legA* promoter. The accumulation with the *arc5l* promoter construct is thus 2 to 3 hundred times higher than the reported levels with the *legA* promoter. As we only obtained one transgenic plant line, most probably plant lines with even higher scFv levels can be obtained. Goossens *et al*. (1999) already obtained 4 times higher levels of ARC5l protein in homozygous seed stocks compared to segregating seed stocks and this under control of the same *arc5l* promoter. As such, after obtaining more transgenic lines and selection of homozygous lines, we expect to reach 10% scFv levels in *Phaseolus acutifolius*, by using this *arc5l* promoter construct.

### References

Bustos, M.M., Begum, D., Kalkan, F.A., Battraw, M.J., and Hall, T.C. (1991). Positive and negative cis-acting DNA domains are required for spatial and temporal regulation of gene expression by a seed storage promoter. *EMBO* 10 (6), 1469-1479.

Clough, S.J., and Bent, A.F. (1998). Floral dip: a simplified method for Agrobacterium-mediated transformation of Arabidopsis thaliana. *Plant J.* 16 (6), 735-743.

De Jaeger, G., Buys, E., Eeckhout, D., De Wilde, C., Jacobs, A., Kapila, J., Angenon, G., Van Montagu, M., Gerats, T., and Depicker, A. High level accumulation of single-chain variable fragments in the cytosol of transgenic *Petunia hybrida* (1999). *Eur. J. Biochem.* 259, 426-434.

Denecke, J., De Rycke, R. and Botterman, J. (1992). Plant and mammalian sorting signals for protein retention in the endoplasmic reticulum contain a conserved epitope. *EMBO J* 11: 2345-2355.

Dillen, W., De Clercq, J., Goossens, A., Van Montagu, M. and Angenon, G. (1997). *Agrobacterium-*mediated transformation of *Phaseolus acutifolius* A. Gray. *Theor. Appl. Genet.* 94, 151-158.

Evan, G.I., Lewis, G.K., Ramsay, G., and Bishop, J.M. (1985). Isolation of monoclonal antibodies specific for Human c-myc Proto-oncogene product. *Mol. And Cell. Biol*. 12, 3610-3616.

Fiedler, U., Phillips, J. Artsaenko, O. and Conrad, U. (1997). Optimization of scFv antibody production in transgenic plants. *Immunotechnology* 3, 205-216

Goddijn, O.J.M. and Pen, J. (1995). Plants as bioreactors. *Trends Biotechnol*. 13, 379-387.

Goossens, A., Geremia, R., Bauw, G., Van Montagu, M. And Angenon, G. (1994). Isolation and characterization of arcelin-5 proteins and cDNAs. *Eur. J. Biochem.* 225*,* 787-795.

Goossens, A., Ardiles Diaz, W., De Keyser, A., Van Montagu, M., and Angenon, G. (1995). Nucleotide sequence of an *arcelin 5-I* genomic clone from wild *Phaseolus vulgaris* (Z50202). *Plant Physiol.* 109, 722, PGR95-075.

Goossens, A., Dillen, W., De Clercq, J., Van Monatgu, M., and Angenon, G. (1999). The *arcelin-5* gene of *Phaseolus vulgaris* directs high seed-specific expression in transgenic *Phaseolus acutifolius* and Arabidopsis plants. *Plant Physiology,* Vol. 120, p. 1095-1104.

Hemming, D. (1995) Molecular farming: using transgenic plants to produce novel proteins and other chemicals. *AgBiotech News Inform*. 7, 19N-29N.

Krebbers, E., Herdies, L., De Clercq, A., Seurinck, J., Leemans, J., Van Damme, J., Segura, M., Gheysen, G., Van Montagu, M. and Vandekerckhove, J. (1988). Determination of the processing sites of an *Arabidopsis* 2S albumin and characterization of the complete gene family. *Plant Physiol* 87: 859-866.

Perrin, Y., Vaquero, C., Gerrard, I., Sack, M., Drossard, J., Stöger, E., Christou, P., and Fischer, R. (2000). Transgenic pea seeds as bioreactors for the production of a single-chain Fv fragment (scFV) antibody used in cancer diagnosis and therapy. *Molecular breeding* 6, 345-352.

Valvekens, D., Van Montagu, M, and Van Lijsebettens, M. (1988). *Agrobacterium tumefaciens*-mediated transformation of *Arabidopsis thaliana* root explants by using kanamycin selection. *PNAS USA* 85, 5536-5540.

van der Geest, A.H.M., Hall, G.E., Spiker, S., and Hall, T.C. (1994). The β-phaseolin gene is flanked by matrix attachment regions. *The Plant Journal* 6(3), 413-423.

Vitale, A. and Bollini, R. (1995) Legume storage proteins. In *Seed development and germination* (Kigel, J. and Galili, G. eds.) p73-102, Marcel Dekker Inc., New York.

Whitelam, G.C., Cockburn, B., Gandecha, A.R. and Owen, M.R.L. (1993). Heterologous protein production in transgenic plants. *Biotechnol. Genet. Eng. Rev.* 11, 1-29.

## Claims

1. A seed preferred expression cassette having gene regulatory elements comprising:
- the arcelin promoter comprising the sequence shown in SEQ ID N° 1,
- the arcelin 5I leader shown in SEQ ID N° 2,
- the 2S2 storage albumin signal peptide shown in SEQ ID N° 4,
- a heterologous gene of interest, and
- the arcelin 5I 3'end comprising the sequence shown in SEQ ID N° 3, wherein said heterologous gene of interest is placed between said leader sequence and said arcelin 5I 3' sequence.

2. A seed preferred expression cassette according to claim 1, whereby said gene of interest is fused the sequence encoding the 2S2 storage albumin signal peptide.

3. A seed preferred expression cassette according to claims 1 or 2, whereby the heterologous gene of interest encodes a single chain Fv fragment.

4. A method to obtain seed preferred expression of a heterologous protein, using an expression cassette according to any of claims 1-3, at a level of at least 10% of the total soluble seed protein, with the proviso that said heterologous protein is not an unmodified seed storage protein.

5. A method according to claim 4 wherein said level is at least 15% of the total soluble seed protein.

6. A plant cell transformed with an expression cassette according to any of the claims 1-3.

7. A transgenic plant comprising an expression cassette according to any of the claims 1-3.

## Patentansprüche

1. Eine für Samen bevorzugte Expressionskassette mit genregulatorischen Elementen, wobei die Expressionskassette das Folgende umfasst:
- den Arcelin-Promotor, umfassend die in SEQ ID NR. 1 gezeigte Sequenz,
- den in SEQ ID NR. 2 gezeigten Leader von Arcelin 5I,
- das in SEQ ID NR. 4 gezeigte Signalpeptid des 2S2-Speicher-Albumins,
- ein heterologes Gen von Interesse und
- das 3'-Ende von Arcelin 5I, umfassend die in SEQ ID NR. 3 gezeigte Sequenz,
wobei das heterologe Gen von Interesse zwischen der Leader-Sequenz und der 3'-Sequenz von Arcelin 5I gelegen ist.

2. Die für Samen bevorzugte Expressionskassette gemäß Anspruch 1, wobei das heterologe Gen von Interesse mit der Sequenz fusioniert ist, die das Signalpeptid des 2S2-Speicher-Albumins kodiert.

3. Die für Samen bevorzugte Expressionskassette gemäß Anspruch 1 oder 2, wobei das heterologe Gen von Interesse ein Einzelketten-Fv-Fragment kodiert.

4. Verfahren unter Verwendung einer Expressionskassette gemäß einem der Ansprüche 1-3, um eine für Samen bevorzugte Expression eines heterologen Proteins mit einer Konzentration von wenigstens 10% des gesamten löslichen Proteins des Samens zu erhalten, unter der Voraussetzung, dass das heterologe Protein kein unmodifiziertes Speicherprotein aus Samen ist.

5. Verfahren gemäß Anspruch 4, wobei die Konzentration wenigstens 15% des gesamten löslichen Proteins des Samens ist.

6. Eine mit einer Expressionskassette gemäß einem der Ansprüche 1-3 transformierte Pflanzenzelle.

7. Eine transgene Pflanze, umfassend eine Expressionskassette gemäß einem der Ansprüche 1-3.

## Revendications

1. Cassette d'expression préférée dans une semence, ayant des éléments de régulation des gènes, comprenant :
- le promoteur arceline comprenant la séquence représentée dans la SEQ ID N°1,
- la séquence de tête d'arceline 5I représentée dans la SEQ ID N°2,
- le peptide signal 2S2 de stockage d'albumine représenté dans la SEQ ID N°4,
- un gène hétérologue d'intérêt, et
- l'extrémité 3' d'arceline 5I, comprenant la séquence représentée dans la SEQ ID N°3, où ledit gène hétérologue d'intérêt est placé entre la séquence de tête et la séquence 3' d'arceline 5I.

2. Cassette d'expression préférée dans une semence selon la revendication 1, dans laquelle ledit gène hétérologue d'intérêt est fusionné à la séquence codant pour le peptide signal 2S2 de stockage d'albumine.

3. Cassette d'expression préférée dans une semence selon la revendication 1 ou 2, dans laquelle ledit gène hétérologue d'intérêt code pour un fragment Fv à chaine simple.

4. Procédé d'obtention de l'expression préférée dans une semence d'une protéine hétérologue, à l'aide d'une cassette d'expression selon l'une quelconque des revendications 1 à 3, à un taux d'au moins 10% des protéines solubles totales de la semence, avec la condition que ladite protéine hétérologue ne soit pas une protéine de stockage de semence, non modifiée.

5. Procédé selon la revendication 4, dans lequel ledit taux est d'au moins 15% des protéines solubles totales de la semence.

6. Cellule végétale transformée avec une cassette d'expression selon l'une quelconque des revendications 1 à 3.

7. Plante transgénique comprenant une cassette d'expression selon l'une quelconque des revendications 1 à 3.


### SEQUENCE LISTING

<110> Vlaams Interuniversitair Instituut voor Biotechnol
<120> Heterologous gene expression in plants
<130> GAN/ARC/V064
<140>
   <141>
<150> 00202278.8
   <151> 2000-06-29
<160> 5
<170> PatentIn Ver. 2.1
<210> 1
   <211> 1821
   <212> DNA
   <213> Phaseolus vulgaris
<400> 1
   gtagacaaaa tcccatcttt tcctacataa ttcttctaca gttaaccttc aaatcatatt 60
   ttcattattc acaaatatct agtcattcat acgaataaat atatattttt ttcacataca 120
   attatgataa tatattaaaa agtgaacttt aaatttaatt taatcttata aaatcaactt 180
   ataaaatgag atttctacct acgattaata aaaataactt tgatatcata ttaaaaaata 240
   aactttaaac ctaactcaac tttataaaac caatttataa aataaaattt acactcagtt 300
   atgaattata aaatgaaata gtttttaggt gacgtggaat ctccatccga ttaatcaata 360
   tttgggtgat gttattgtta ttatagaaac taaaaacatg ccaaataatt tacaatatat 420
   agattcagtt aaatcaattc agcttgtctc cttgactaat aaaaaaaaac tttagactat 480
   tattcagatt tacacttcat ctctcatgat atccctcaaa gtgaatttca ttcatggcac 540
   catttatata atcaacaatt ttaaaaatat gcaaatttgt accagtaaat gctttaatgt 600
   ccctgataaa cacaaaaaaa aaaaaattca tatttttttc ttattaaata aagaagttca 660
   ttgtaagaga aattaggatc cttcaataga aaatgtgtta tttcctcatc accagacaaa 720
   ggggcaacag ttaacaaaac aaatttatgt ttcatttgag attaaggaag gtaaggaaga 780
   aaaaagatta aaaaaaatgt ccttatctct ttgtttctgt aataataata taagagactt 840
   aaacttttaa tataataatt gtaattaggt tttctagtca tgagcaccac tcagagacaa 900
   gatttcaaga aaacaatttt gttaaacatc ttattagaaa cttttagtta agtcttgaag 960
   ttagaattaa acaaaaaaaa gtacacacga gaaacacaat aaacccacta ccgtcaggtt 1020
   atcataagga tgaaatgttt tgatatcatt aaatataaca cacacaaaaa tacatctaat 1080
   tataacaata tatgttatac atatattttt gtaaaaactt agagtttttc aaaacattct 1140
   aatacatgat tagagtttat agaaatacaa atatttaaaa aatataattt taaaaaaaca 1200
   ttctaaagtc attcagatcc tctcacacct gtgtgatcat ttagtcatgt atgtagtaca 1260
   atcattgtag ttcacaacag agtaaaataa ataaggataa actagggaat atatataata 1320
   tatacaatta aataaaaaag ggaaaatcaa attagaattt ttgattcccc acatgacaca 1380
   actcaccatg cacgctgcca cctcagctcc ctcctctcca cacatgtctc atgtcacttt 1440
   cgactttggt tttttcacta tgacacaact cgccatgcat gttgccacgt gagctccttc 1500
   ctcttcccat gatgacacca ctgggcatgc atgctgccac ctcagctccc acctcttctc 1560
   attatgagcc tactggccat gcacactgcc acctcagcac tcctctcact tcccattgct 1620
   acctgccaaa ccgcttctct ccataaatat ctatttaaat ttaaactaat tatttcatat 1680
   acttttttga tgacgtggat gcattgccat cgttgtttaa taattgttaa tttggagttg 1740
   aataataaaa tgaaagaaaa aagttggaaa gattttgcat ttgttgttgt ataaatagag 1800
   aagagagtga tggttaatgc a 1821
<210> 2
   <211> 13
   <212> DNA
   <213> Phaseolus vulgaris
<400> 2
   tgaatgcatg atc 13
<210> 3
   <211> 1280
   <212> DNA
   <213> Phaseolus vulgaris
<400> 3
   actcccaaaa ccaccttccc tgtgacagtt aaaccctgct tatacctttc ctcctaataa 60
   tgttcatctg tcacacaaac taaaataaat aaaatgggag caataaataa aatgggagct 120
   catatattta caccatttac actgtctatt attcaccatg ccaattatta cttcataatt 180
   ttaaaattat gtcattttta aaaattgctt aatgatggaa aggattatta taagttaaaa 240
   gtataacata gataaactaa ccacaaaaca aatcaatata aactaactta ctctcccatc 300
   taatttttat ttaaatttct ttacacttct cttccatttc tatttctaca acattattta 360
   acatttttat tgtatttttc ttactttcta actctattca tttcaaaaat caatatatgt 420
   ttatcaccac ctctctaaaa aaaactttac aatcattggt ccagaaaagt taaatcacga 480
   gatggtcatt ttagcattaa aacaacgatt cttgtatcac tatttttcag catgtagtcc 540
   attctcttca aacaaagaca gcggctatat aatcgttgtg ttatattcag tctaaaacaa 600
   ttgttatggt aaaagtcgtc attttacgcc tttttaaaag atataaaatg acagttatgg 660
   ttaaaagtca tcatgttaga tcctccttaa agatataaaa tgacagtttt ggataaaaag 720
   tggtcatttt atacgctctt gaaagatata aaacgacggt tatggtaaaa gctgccattt 780
   taaatgaaat atttttgttt tagttcattt tgtttaatgc taatcccatt taaattgact 840
   tgtacaatta aaactcaccc acccagatac aatataaact aacttactct cacagctaag 900
   ttttatttaa atttctttac acttcttttc catttctatt tctatgacat taactaacat 960
   ttttctcgta attttttttc ttattttcta actctatcca tttcaaatcg atatatgttt 1020
   atcaccacca ctttaaaaag aaaatttaca atttctcgtg caaaaaagct aaatcatgac 1080
   cgtcatttta gcattaaaac aacgattctt gtatcgttgt ttttcagcat gtagtccatt 1140
   cttttcaagc aaagacaaca gctatataat catcgtgtta tattcagtct aaaacaacag 1200
   taatgataaa agtcatcatt ttaggccttt ctgaaatata tagaacgaca ttcatggtaa 1260
   aaaatcgtca ttttagatcc                                             1280
<210> 4
   <211> 63
   <212> DNA
   <213> Arabidopsis thaliana
<400> 4
   atggcaaaca agctcttcct cgtctgcgca actttcgccc tctgcttcct cctcaccaac 60
   gcc                                                               63
<210> 5
   <211> 1415
   <212> DNA
   <213> Phaseolus vulgaris
<400> 5
   ggtcgacggt atcgataagc ttgatatcga attcctgcag cccaattcat tgtactccca 60
   gtatcattat agtgaaagtt ttggctctct cgccggtggt tttttacctc tatttaaagg 120
   ggttttccac ctaaaaattc tggtatcatt ctcactttac ttgttacttt aatttctcat 180
   aatctttggt tgaaattatc acgcttccgc acacgatatc cctacaaatt tattatttgt 240
   taaacatttt caaaccgcat aaaattttat gaagtcccgt ctatctttaa tgtagtctaa 300
   cattttcata ttgaaatata taatttactt aattttagcg ttggtagaaa gcataatgat 360
   ttattcttat tcttcttcat ataaatgttt aatatacaat ataaacaaat tctttacctt 420
   aagaaggatt tcccatttta tattttaaaa atatatttat caaatatttt tcaaccacgt 480
   aaatctcata ataataagtt gtttcaaaag taataaaatt taactccata atttttttat 540
   tcgactgatc ttaaagcaac acccagtgac acaactagcc atttttttct ttggataaaa 600
   aaatccaatt atcattgtat tttttttata caatgaaaat ttcaccaaac aatcatttgt 660
   ggtatttctg aagcaagtca tgttatgcaa aattctataa ttcccatttg acactacgga 720
   agtaactgaa gatctgcttt tacatgcgag acacatcttc taaagtaatt ttaataatag 780
   ttactatatt caagatttca tatatcaaat actcaatatt acttctaaaa aattaattag 840
   atataattaa aatattactt ttttaatttt aagtttaatt gttgaatttg tgactattga 900
   tttattattc tactatgttt aaattgtttt atagatagtt taaagtaaat ataagtaatg 960
   tagtagagtg ttagagtgtt accctaaacc ataaactata acatttatgg tggactaatt 1020
   ttcatatatt tcttattgct tttacctttt cttggtatgt aagtccgtaa ctagaattac 1080
   tgtgggttgc catggcactc tgtggtcttt tggttcatgc atggatgctt gcgcaagaaa 1140
   aagacaaaga acaaagaaaa aagacaaaac agagagacaa aacgcaatca cacaaccaac 1200
   tcaaattagt cactggctga tcaagatcgc cgcgtccatg tatgtctaaa tgccatgcaa 1260
   agcaacacgt gcttaacatg cactttaaat ggctcaccca tctcaaccca cacacaaaca 1320
   cattgccttt ttcttcatca tcaccacaac cacctgtata tattcattct cttccgccac 1380
   ctcaatttct tcacttcaac acacgtcaac ctgca                            1415